(19) Europäisches Patentamt
European Patent Office
Office européen des brevets

(11) EP 4 494 696 A1

(12) EUROPEAN PATENT APPLICATION
published in accordance with Art. 153(4) EPC

(43) Date of publication:
**22.01.2025 Bulletin 2025/04**

(21) Application number: **23791922.0**

(22) Date of filing: **20.04.2023**

(51) International Patent Classification (IPC):
***A61P 1/02*** (2006.01) ***A61Q 3/00*** (2006.01)

(52) Cooperative Patent Classification (CPC):
**A23L 29/206; A23L 29/269; A23L 29/288; A23L 33/10; A61K 8/19; A61K 8/24; A61K 8/35; A61K 8/36; A61K 8/365; A61K 8/73; A61K 9/06; A61K 9/08; A61K 31/12; A61K 31/121; A61K 31/19;** (Cont.)

(86) International application number:
**PCT/JP2023/015811**

(87) International publication number:
**WO 2023/204274 (26.10.2023 Gazette 2023/43)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **21.04.2022 JP 2022070188**
**27.12.2022 JP 2022210329**

(71) Applicants:
- **Sanyo Chemical Industries, Ltd.**
**Kyoto-shi, Kyoto 605-0995 (JP)**
- **Joycle Co., Ltd.**
**Kyotanabe-shi, Kyoto 610-0323 (JP)**

(72) Inventors:
- **SUZUKI, Ryosuke**
**Kyoto-shi, Kyoto 605-0995 (JP)**
- **HIRAO, Naoya**
**Kyoto-shi, Kyoto 605-0995 (JP)**
- **OTA, Koji**
**Kyoto-shi, Kyoto 605-0995 (JP)**
- **HORIE, Seiji**
**Kyoto-shi, Kyoto 605-0995 (JP)**
- **MIYAZAKI, Koji**
**Kyotanabe-shi, Kyoto 610-0323 (JP)**

(74) Representative: **Manitz Finsterwald Patent- und Rechtsanwaltspartnerschaft mbB**
**Martin-Greif-Strasse 1**
**80336 München (DE)**

# (54) MICROBIOTA IMPROVING AGENT

(57) The present invention aims to provide a microbiota improving agent for improving at least one of mucosal microbiota, skin microbiota, or nail microbiota, the microbiota improving agent exhibiting a high pathogenic bacteria inhibitory effect while maintaining resident non-pathogenic bacteria, thus persistently improving the microbiota balance. The present invention relates to a microbiota improving agent for improving at least one of mucosal microbiota, skin microbiota, or nail microbiota, the microbiota improving agent containing: a substance (X); and a base (Y) containing a non-volatile component, the substance (X) including at least one selected from the group consisting of a compound (X1) having two or more hydroxy groups contained in at least one oxoacid group and a salt of the compound (X1), a compound (X2) having one hydroxy group contained in an oxoacid group and a salt of the compound (X2), a heterocyclic compound (X3) having no oxoacid group and a salt of the compound (X3), and a ketone (X4) having no oxoacid group. In the compound (X1), a total mass percentage of the at least one oxoacid group, a hydroxy group not contained in the oxoacid group, a carbonyl group not contained in the oxoacid group, carbon atoms contained in an imidazole skeleton and a pyridine skeleton, and nitrogen atoms contained in the imidazole skeleton and the pyridine skeleton is 45% by mass or more based on a molecular weight of the compound (X1). The compound (X2) has an atom having an unshared electron pair, the atom excluding atoms contained in the oxoacid group and atoms contained in an amino group not constituting an imidazole skeleton and in an amino group not constituting a pyridine skeleton. In the compound (X2), a total mass percentage of the oxoacid group, a hydroxy group not contained in the oxoacid group, a carbonyl group not contained in the oxoacid group, carbon atoms contained in an imidazole skeleton

(Cont. next page)



Processed by Luminess, 75001 PARIS (FR)

and a pyridine skeleton, and nitrogen atoms contained in the imidazole skeleton and the pyridine skeleton is 55% by mass or more based on a molecular weight of the compound (X2). The heterocyclic compound (X3) has an imidazole skeleton or a pyridine skeleton and at least one of a hydroxy group or a carbonyl group, or has two or more skeletons selected from the group consisting of an imidazole skeleton and a pyridine skeleton. The ketone (X4) has a linear β-diketone structure. The substance (X) has a molecular weight of 550 or less. The non-volatile component has an HLB value of 7 or more. The microbiota improving agent satisfies the following conditions (i) and (ii): (i) the microbiota improving agent has a worked penetration of 100 to 400 as measured in accordance with JIS K 2220; and (ii) the microbiota improving agent has a viscosity at 37°C of 1 to 4,000 Pa·s as measured with a cone-and-plate rheometer.

(52) Cooperative Patent Classification (CPC): (Cont.)
**A61K 33/00; A61K 33/24; A61K 33/42; A61K 47/38; A61P 1/02; A61Q 3/00; A61Q 11/00; A61Q 19/00**

## Description

TECHNICAL FIELD

The present invention relates to microbiota improving agents.

BACKGROUND ART

Various bacteria coexist symbiotically in the human body (e.g., the mucosa (oral mucosa, etc.), skin, nails), and bacteria form a bacterial community called microbiota. The microbiota includes a mixture of so-called resident non-pathogenic bacteria and pathogenic bacteria.

The microbiota balance is usually maintained, which is known as a healthy state, and diseases caused by pathogenic bacteria are suppressed.

However, if the microbiota balance is disrupted due to factors such as diet, antibiotic intake, stress, aging, or poor hygiene, the numbers of pathogenic bacteria and opportunistic infectious bacteria increase, causing diseases. Therefore, in order to suppress diseases, it is important not only to inhibit pathogenic bacteria but also to maintain the healthy microbiota balance between pathogenic bacteria and resident non-pathogenic bacteria.

Known techniques relating to a pathogenic factor production inhibitor that exhibits a function of acting on specific harmful bacteria include a technique disclosed in Patent Literature 1.

CITATION LIST

- Patent Literature

Patent Literature 1: JP 5716173 B

SUMMARY OF INVENTION

- Technical Problem

However, as a result of investigation, it was found that the pathogenic factor production inhibitor described in Patent Literature 1 when used as is not only prevents or reduces the action of pathogenic bacteria, but also has a significant adverse effect on resident non-pathogenic bacteria. It is also difficult for the inhibitor to persistently prevent or reduce the action of pathogenic bacteria on the application site. Such a pathogenic factor production inhibitor is required to improve in order to persistently maintain the healthy microbiota balance.

Therefore, the present invention aims to provide a microbiota improving agent for improving at least one of mucosal microbiota, skin microbiota, or nail microbiota, the microbiota improving agent exhibiting a high pathogenic bacteria inhibitory effect while maintaining resident non-pathogenic bacteria, thus persistently improving the microbiota balance.

- Solution to Problem

The present inventors conducted extensive research to solve the above problems and arrived at the present invention. Specifically, the present invention relates to a microbiota improving agent for improving at least one of mucosal microbiota, skin microbiota, or nail microbiota, the microbiota improving agent containing:

a substance (X); and
a base (Y) containing a non-volatile component,
the substance (X) including at least one selected from the group consisting of
a compound (X1) having two or more hydroxy groups contained in at least one oxoacid group and a salt of the compound (X1),
a compound (X2) having one hydroxy group contained in an oxoacid group and a salt of the compound (X2),
a heterocyclic compound (X3) having no oxoacid group and a salt of the compound (X3), and
a ketone (X4) having no oxoacid group,
wherein in the compound (X1), a total mass percentage of the at least one oxoacid group, a hydroxy group not contained in the oxoacid group, a carbonyl group not contained in the oxoacid group, carbon atoms contained in an imidazole skeleton and a pyridine skeleton, and nitrogen atoms contained in the imidazole skeleton and the pyridine skeleton is 45% by mass or more based on a molecular weight of the compound (X1),

the compound (X2) has an atom having an unshared electron pair, the atom excluding atoms contained in the oxoacid group and atoms contained in an amino group not constituting an imidazole skeleton and in an amino group not constituting a pyridine skeleton,
in the compound (X2), a total mass percentage of the oxoacid group, a hydroxy group not contained in the oxoacid group, a carbonyl group not contained in the oxoacid group, carbon atoms contained in an imidazole skeleton and a pyridine skeleton, and nitrogen atoms contained in the imidazole skeleton and the pyridine skeleton is 55% by mass or more based on a molecular weight of the compound (X2),
the heterocyclic compound (X3) has an imidazole skeleton or a pyridine skeleton and at least one of a hydroxy group or a carbonyl group, or has two or more skeletons selected from the group consisting of an imidazole skeleton and a pyridine skeleton,
the ketone (X4) has a linear β-diketone structure,
the substance (X) has a molecular weight of 550 or less,
the non-volatile component has an HLB value of 7 or more, and
the microbiota improving agent satisfies the following conditions (i) and (ii):

(i) the microbiota improving agent has a worked penetration of 100 to 400 as measured in accordance with JIS K 2220; and
(ii) the microbiota improving agent has a viscosity at 37°C of 1 to 4,000 Pa·s as measured with a cone-and-plate rheometer.

- Advantageous Effects of Invention

The present invention provides a microbiota improving agent for improving at least one of mucosal microbiota, skin microbiota, or nail microbiota, the microbiota improving agent exhibiting a high pathogenic bacteria inhibitory effect while maintaining resident non-pathogenic bacteria, thus persistently improving the microbiota balance.

DESCRIPTION OF EMBODIMENTS

The microbiota improving agent of the present invention for improving at least one of mucosal microbiota, skin microbiota, or nail microbiota (hereinafter also referred to as "microbiota improving agent of the present invention") contains a substance (X) and a base (Y) containing a non-volatile component, the substance (X) including at least one selected from the group consisting of a compound (X1) having two or more hydroxy groups contained in at least one oxoacid group and a salt of the compound (X1), a compound (X2) having one hydroxy group contained in an oxoacid group and a salt of the compound (X2), a heterocyclic compound (X3) having no oxoacid group and a salt of the compound (X3), and a ketone (X4) having no oxoacid group.
The microbiota improving agent of the present invention exhibits a high pathogenic bacteria inhibitory effect while maintaining resident non-pathogenic bacteria, thus improving the microbiota balance.
Here, the "high pathogenic bacteria inhibitory effect" means at least one of killing harmful bacteria, inactivating harmful bacteria and inhibiting their growth, or inhibiting harmful bacteria from producing toxic substances (pathogenic factors).
The term "pathogenic bacteria" herein refers to bacteria, fungi, and the like that are highly pathogenic and cause various infectious diseases and the like as they grow. Specific examples thereof include *Staphylococcus aureus, Streptococcus pyogenes, Pseudomonas aeruginosa, Porphyromonas gingivalis, Fusobacterium nucleatum, Prevotella intermedia, Trichophyton rubrum, Trichophyton mentagrophytes, Candida albicans,* and *Candida glabrata.*
The term "resident non-pathogenic bacteria" refers to resident bacteria that inhibit the growth of pathogenic bacteria and maintain health. Specific examples thereof include *Staphylococcus epidermidis* and *Streptococcus salivarius.*
Of the above-listed pathogenic bacteria, *Porphyromonas gingivalis, Fusobacterium nucleatum,* and *Prevotella intermedia* are known as pathogenic bacteria that can occur in the oral cavity.
Of the above-listed pathogenic bacteria, *Trichophyton rubrum, Trichophyton mentagrophytes, Staphylococcus aureus, Candida albicans,* and *Candida glabrata* are known as pathogenic bacteria that can occur in the skin and nails.
Of the above-listed non-pathogenic bacteria, *Streptococcus salivarius* is known as a resident bacteria present in the oral cavity.
Of the above-listed non-pathogenic bacteria, *Staphylococcus epidermidis* is known as a resident bacteria present in the skin and nails.
The microbiota improving agent of the present invention can selectively inhibit the quorum sensing mechanism (i.e., the mechanism that produces autoinducers) of pathogenic bacteria, and has a high pathogenic bacteria inhibitory effect while maintaining resident non-pathogenic bacteria.

Substance (X)

The microbiota improving agent of the present invention contains a substance (X).

The substance (X) includes at least one selected from the group consisting of a compound (X1) having two or more hydroxy groups contained in at least one oxoacid group and a salt of the compound (X1), a compound (X2) having one hydroxy group contained in an oxoacid group and a salt of the compound (X2), a heterocyclic compound (X3) having no oxoacid group and a salt of the compound (X3), and a ketone (X4) having no oxoacid group.

The substance (X) may include two or more of the compounds (X1), two or more of the compounds (X2), two or more of the heterocyclic compounds (X3), or two or more of the ketones (X4). The substance (X) may include two or more salts of the compound(s) (X1), two or more salts of the compound(s) (X2), or two or more salts of the compound(s) (X3).

The compound (X1) has two or more hydroxy groups contained in at least one oxoacid group. The compound (X1) may have two or more oxoacid groups.

Herein, the term "oxoacid group" refers to a group having a structure in which a hydroxy group (-OH) and an oxo group (=O) are bonded to a single central atom (such as a carbon atom, a phosphorus atom, a sulfur atom, or a tungsten atom). One oxoacid group may contain two or more hydroxy groups or two or more oxo groups.

For example, triphosphate, which is represented by the following formula:

[Chem. 1]

$$HO-P(=O)(OH)-O-P(=O)(OH)-O-P(=O)(OH)-OH$$

,

contains two different oxoacid groups, i.e., oxoacid groups each represented by $-P(=O)(OH)_2$ at both ends of the molecule and an oxoacid group represented by -P(=O) (OH)- in the center of the molecule. The oxoacid groups at both ends each contain two hydroxy groups.

From the viewpoint of the effect of the microbiota improving agent, in the compound (X1), the total mass percentage of the at least one oxoacid group, a hydroxy group not contained in the oxoacid group, a carbonyl group (C=O) not contained in the oxoacid group, carbon atoms contained in an imidazole skeleton and a pyridine skeleton, and nitrogen atoms contained in the imidazole skeleton and the pyridine skeleton is 45% by mass or more, preferably 55% by mass or more, based on the molecular weight of the compound (X1). When the compound (X1) in the microbiota improving agent includes multiple compounds, each compound satisfies this condition. Herein, the mass of an oxoacid group is the total mass of the central atom, the oxo group(s), and the hydroxy group(s).

For example, malic acid, which has a molecular weight of 134.1 and is represented by the following formula:

[Chem. 2]

$$HOOC-CH_2-CH(OH)-COOH$$

,

has two carboxy groups (formula weight 45.0), which are oxoacid groups, and one hydroxy group (formula weight 17.0) not contained in the oxoacid groups. The total mass percentage of these groups based on the molecular weight of malic acid is calculated as follows:

$$[(45.0 \times 2 + 17.0 \times 1)/134.1] \times 100 = 80 \text{ (\% by mass)}.$$

The compound (X1) has at least one oxoacid group as an essential group, and optionally has a hydroxy group not contained in the oxoacid group, a carbonyl group not contained in the oxoacid group, an imidazole skeleton, and a pyridine skeleton as non-essential components.

Herein, the "imidazole skeleton" refers to a structure represented by the following formula:

[Chem. 3]

N
N
R

wherein R is a hydrogen atom or a group capable of bonding to imidazole.

The "pyridine skeleton" refers to a structure represented by the following formula:

[Chem. 4]

N

These structures may be contained in a fused ring.

From the viewpoint of a further improvement in the effect of the microbiota improving agent, in the compound (X1), the total mass percentage of the at least one oxoacid group, a hydroxy group not contained in the oxoacid group, and a carbonyl group not contained in the oxoacid group is 45% by mass or more based on the molecular weight of the compound (X1).

Examples of the compound (X1) include polyphosphoric acid, tungstic acid ($H_2WO_4$), ethylenediaminetetraacetic acid, glutamic acid, iminodiacetic acid, malic acid, citric acid, tartaric acid, adenosine triphosphate, and guanosine triphosphate. Herein, tungstic acid is considered to be a compound having two hydroxy groups contained in an oxoacid group, as shown in the following chemical formula (1), and is thus included in the compound (X1).

[Chem. 5]

O=W(=O)(OH)OH (1)

From the viewpoint of the effect of the microbiota improving agent, preferably, the compound (X1) has no "-NH2" group.

When the compound (X1) is a hydroxycarboxylic acid, the ratio of the number of moles of hydroxy groups to the number of moles of carboxy groups (-COOH) (number of moles of -OH/number of moles of -COOH) in the hydroxycarboxylic acid is preferably 1/3 or more, more preferably 1/2 or more, from the viewpoint of the effect of the microbiota improving agent.

Non-limiting examples of the salt of the compound (X1) include inorganic acid salts such as hydrochlorides, sulfates, nitrates, and phosphates; organic acid salts such as acetates, citrates, maleates, malates, oxalates, lactates, succinates, fumarates, and propionates; alkali metal salts such as sodium salts and potassium salts; alkaline earth metal salts such as calcium salts and magnesium salts; ammonium salts; salts with organic bases such as triethylamine, triethanolamine, and pyridine; and amino acid salts with basic amino acids (e.g., arginine) or with acidic amino acids (e.g., glutamic acid). Of these, sodium salts and potassium salts are preferred.

The compound (X2) has an atom having an unshared electron pair, the atom excluding the atoms contained in the oxoacid group and the atoms contained in an amino group not constituting an imidazole skeleton and in an amino group not constituting a pyridine skeleton.

Preferred examples of the atom having an unshared electron pair include the oxygen atom contained in a carbonyl group, the oxygen atom contained in a hydroxy group (excluding the oxygen atoms contained in an oxoacid

group), the nitrogen atom contained in an imidazole skeleton, and the nitrogen atom contained in a pyridine skeleton.

From the viewpoint of the effect of the microbiota improving agent, in the compound (X2), the total mass percentage of the oxoacid group, a hydroxy group not contained in the oxoacid group, a carbonyl group not contained in the oxoacid group, carbon atoms contained in an imidazole skeleton and a pyridine skeleton, and nitrogen atoms contained in the imidazole skeleton and the pyridine skeleton is 55% by mass or more based on the molecular weight of the compound (X2). When the compound (X2) in the microbiota improving agent includes multiple compounds, each compound satisfies this condition.

The compound (X2) has an oxoacid group as an essential group, and optionally has a hydroxy group not contained in the oxoacid group, a carbonyl group not contained in the oxoacid group, an imidazole skeleton, and a pyridine skeleton.

Examples of the compound (X2) include a compound (X21) having no amino group and a compound (X22) having an amino group.

Examples of the compound (X21) having no amino group include hydroxycarboxylic acids (gluconic acid, glucuronic acid, etc.) and keto acids (levulinic acid, etc.).

Examples of the compound (X22) having an amino group include glycylglycine and histidine.

Examples of the salt of the compound (X2) include those described as the salt of the compound (X1), and the same applies to preferred examples thereof.

The heterocyclic compound (X3) has an imidazole skeleton or a pyridine skeleton and at least one of a hydroxy group or a carbonyl group, or has two or more skeletons selected from the group consisting of an imidazole skeleton and a pyridine skeleton.

Examples of the heterocyclic compound (X3) include 8-quinolinol (a compound having one pyridine skeleton and one hydroxy group) and phenanthroline (a compound having two pyridine skeletons).

From the viewpoint of the effect of the microbiota improving agent, in the heterocyclic compound (X3), the total mass percentage of the carbon atoms contained in the imidazole skeleton and the pyridine skeleton, the nitrogen atoms contained in the imidazole skeleton and the pyridine skeleton, the hydroxy group(s), and the carbonyl group(s) is preferably 50% by mass or more based on the molecular weight of the heterocyclic compound (X3). When the heterocyclic compound (X3) in the microbiota improving agent includes multiple compounds, each compound preferably satisfies this condition.

Examples of the salt of the heterocyclic compound (X3) include those described as the salt of the compound (X1).

The ketone (X4) has a linear β-diketone structure.

The ketone (X4) may be a salt having a counter ion.

Examples of the ketone (X4) include benzoylacetone and acetylacetone.

From the viewpoint of the effect of the microbiota improving agent, in the ketone (X4), the total mass percentage of the carbonyl groups is preferably 30% by mass or more based on the molecular weight of the ketone (X4) (in the case where the ketone (X4) is a salt having a counter ion, it is based on the mass of only an ion having a linear β-diketone structure excluding the counter ion). When the ketone (X4) in the microbiota improving agent includes multiple compounds, each compound preferably satisfies this condition.

From the viewpoint of safety, the substance (X) preferably includes at least one selected from the group consisting of levulinic acid and salts thereof, tungstic acid and salts thereof, benzoylacetone, polyphosphoric acid and salts thereof, and acetylacetone. Polyphosphoric acid (the number of repeating phosphate units is preferably 2 to 5, more preferably 3 to 5) and salts thereof are more preferred, and pentasodium triphosphate is particularly preferred.

From the viewpoint of the effect of the microbiota improving agent, the substance (X) has a molecular weight of 550 or less. When the substance (X) in the microbiota improving agent includes multiple compounds, each compound has a molecular weight of 550 or less.

As for the substance (X), the amount of magnesium captured per 100 g of the substance (X) is preferably 1 g or more, as measured by the ion-selective electrode method.

When the substance (X) is a hydrate, 100 g of the substance (X) refers to 100 g by weight excluding hydration water.

The measurement by the ion-selective electrode method is performed using 2 mM of the substance (X) relative to a 1 mM aqueous $MgCl_2$ solution having a temperature of 20°C and a pH of 7.

Base (Y)

The microbiota improving agent of the present invention contains not only the substance (X) but also a base (Y) containing a non-volatile component. The substance (X) does not correspond to a component contained in the base (Y).

The non-volatile component in the base (Y) has an HLB value of 7 or more. If the HLB value of the non-volatile component is less than 7, the spreadability of the microbiota improving agent when applied to the affected area is poor. The HLB value of the non-volatile component is preferably 7 to 60 from the viewpoint of spreadability. A component having an

HLB value of 7 or more in the microbiota improving agent is regarded as the non-volatile component in the base (Y).

The HLB value is an index showing the balance between hydrophilicity and lipophilicity. The HLB value is known as a value calculated by the Oda method described in, for example, Takehiko Fujimoto, "Introduction to Surfactants (Kaimen Kakuseizai Nyumon)", Sanyo Chemical Industries, Ltd., 2007, p. 212, and is not a value calculated by the Griffin method.

The HLB value of a compound as a non-volatile component can be calculated based on the ratio between the organic value and the inorganic value of the compound by the following formula:

$$HLB = 10 \times \text{inorganic value}/\text{organic value}.$$

Regarding the organic values and the inorganic values for giving HLB values, the organic values are set to 20 per carbon atom, and the inorganic values are calculated using the values shown in the table on page 213 of the above-described *"Introduction to Surfactants* (*Kaimen Kakuseizai Nyumon*)" ("Value" of Inorganic group or "Value" of "Inorganic value" of Organic and inorganic group). Examples of the calculation include:

-$CH_3$ group: organic value 20, inorganic value 0;
-$CH_2$- group: organic value 20, inorganic value 0;
=$CH_2$ group: organic value 20, inorganic value 1;
=CH- group: organic value 10, inorganic value 1;
benzene ring: organic value 120, inorganic value 15;
-O- group: inorganic value 20;
-COO-: organic value 20, inorganic value 60;
-OH group: inorganic value 100; and
-COOH group: organic value 20, inorganic value 150.

In the present invention, the HLB values are calculated using the following organic values and inorganic values for the following components.

$COO^-M^+$: organic value 20, inorganic value 400 ("$M^+$" is a counter ion of "$COO^-$" and represents a metal cation or ammonium cation)
Si atom: organic value 0, inorganic value 0

When the non-volatile component includes multiple components, the HLB value of the non-volatile component is determined by the weighted average of the HLB values of the components based on the weight percentages of the components.

The "non-volatile component" in the present invention refers to the residue remaining after a sample is heated and dried in an uncovered glass petri dish at 130°C for 45 minutes in a circulating air dryer.

From the viewpoint of the effect of the microbiota improving agent, the amount (weight percentage) of the non-volatile component is preferably 0.1 to 80% by weight, more preferably 1 to 70% by weight, based on the weight of the microbiota improving agent.

Furthermore, from the viewpoint of the effect of the microbiota improving agent, the amount (weight percentage) of the non-volatile component is preferably 30 to 6,000% by weight, more preferably 50 to 5,000% by weight, relative to the weight of the substance (X).

The non-volatile component in the base (Y) is preferably a polymer having a weight average molecular weight of 10,000 to 3,000,000. Furthermore, the polymer having a weight average molecular weight of 10,000 to 3,000,000 preferably contains a compound having a molecular weight of 500 or more. A polymer contained in the microbiota improving agent and having a weight-average molecular weight of 10,000 to 3,000,000 and an HLB value of 7 or more is regarded as the non-volatile component in the base (Y).

The weight-average molecular weight herein can be measured by gel permeation chromatography (GPC) under the following conditions.

<GPC measurement conditions>

(1) Apparatus: gel permeation chromatography [model number "HLC-8120GPC" available from Tosoh Corporation]
(2) Column: "TSKgel G6000PWxl" and "TSKgel G3000PWxl" connected in series, both available from Tosoh

Corporation
(3) Eluent: a solution prepared by dissolving 0.5% by weight sodium acetate in methanol/water = 30/70 (volume ratio)
(4) Reference substance: polyethylene glycol (hereinafter abbreviated as PEG)
(5) Injection conditions: sample concentration of 0.25% by weight, column temperature of 40°C

Examples of monomers to form the polymer include monosaccharides such as α-glucose, β-glucose, and uronic acids (e.g., mannuronic acid, guluronic acid); (meth)acrylic monomers such as C3-C10 (meth)acrylic monomers (e.g., acrylic acid); (alkyl)alkoxysilanes such as C4-C40 dialkyldialkoxysilanes (e.g., diethoxydimethylsilane, dimethoxydimethylsilane); and alkylene oxides such as C2-C4 alkylene oxides (e.g., ethylene oxide, propylene oxide, butylene oxide).
In the present invention, (meth)acrylic refers to acrylic and/or methacrylic.
In the present invention, (alkyl)alkoxysilanes refer to alkoxysilanes and/or alkylalkoxysilanes.
Specific examples of the polymer include carboxyalkylcellulose and salts thereof (e.g., carboxymethylethylcellulose, sodium carboxymethylcellulose), alkylcellulose and salts thereof (e.g., methylcellulose 4000), sodium alginate, xanthan gum, starch-sodium acrylate graft copolymers, polyalkylene glycols such as polyethylene glycols (e.g., PEG 20000), polydimethylsiloxane (A), and an adduct of alkylene oxide with aliphatic alcohol (B).
The non-volatile component may consist of one of the polymers or may include two or more thereof in combination.
For example, the non-volatile component may include the polydimethylsiloxane (A) and the adduct of alkylene oxide with aliphatic alcohol (B) in combination. In this case, the weighted average of the HLB values of the polydimethylsiloxane (A) and the adduct of alkylene oxide with aliphatic alcohol (B) [weighted average based on the weight percentages of polydimethylsiloxane (A) and the adduct of alkylene oxide with aliphatic alcohol (B)] is 7 or more.
The base (Y) may contain only a non-volatile component, or may contain a volatile component in addition to a non-volatile component.
The following describes as an example of the base (Y) a composition containing the polydimethylsiloxane (A) and the adduct of alkylene oxide with aliphatic alcohol (B) as non-volatile components and water as a volatile component (an additive described later).
Examples of the polydimethylsiloxane (A) include those having a structure in which a dimethylsiloxane unit {-$(CH_3)_2SiO$-} is a repeating unit. Specific examples thereof include those represented by the following formula (1):

$$CH_3\text{-}[(CH_3)_2SiO]_n\text{-}Si\text{-}(CH_3)_3 \qquad (1)$$

wherein n is the number-average number of dimethylsiloxane units and is a number from 60 to 500, but is not necessarily an integer.
In the formula (1), n is preferably a number from 100 to 450, more preferably a number from 150 to 400, from the viewpoint of spreadability.
Examples of commercially available products of the polydimethylsiloxane (A) include Dow Corning® Q7-9120 Silicone Fluid, 100 cSt (n = 85), 350 cSt (n = 200), and 1000 cSt (n = 350), available from Dow Corning Toray Co., Ltd.
From the viewpoint of the persistence of the effect of the microbiota improving agent, the polydimethylsiloxane (A) is preferably polydimethylsiloxane represented by the formula (1).
From the viewpoint of spreadability, the adduct of alkylene oxide with aliphatic alcohol (B) is preferably one represented by the following formula (2):

$$R^1\text{-}(OA)_n\text{-}OH \qquad (2)$$

wherein $R^1$ represents a C8-C18 alkyl group or a C8-C18 alkenyl group; OA represents a C2-C4 oxyalkylene group; and m represents the average number of moles of oxyalkylene groups added and is a number from 1 to 50, but is not necessarily an integer.
In the formula (2), $R^1$ represents a C8-C18 alkyl group or a C8-C18 alkenyl group.
The C8-C18 alkyl group may be a linear C8-C18 alkyl group or a branched C8-C18 alkyl group.
Examples of the linear C8-C18 alkyl group include n-octyl, n-decyl, n-undecyl, n-dodecyl, n-tridecyl, n-tetradecyl, n-pentadecyl, n-hexadecyl, n-heptadecyl, and n-octadecyl groups.
Examples of the branched C8-C18 alkyl group include 2-ethylhexyl, isodecyl, isoundecyl, isododecyl, isotridecyl, isotetradecyl, isopentadecyl, isohexadecyl, isoheptadecyl, and isooctadecyl groups.
The C8-C18 alkenyl group may be a linear C8-C18 alkenyl group or a branched C8-C18 alkenyl group.
Examples of the linear C8-C18 alkenyl group include n-octenyl, n-dodecenyl, n-hexadecenyl, and n-octadecenyl groups.
Examples of the branched C8-C18 alkenyl group include isodecenyl, isododecenyl, and isooctadecenyl groups.
From the viewpoint of spreadability, $R^1$ is preferably a linear C8-C18 alkyl group, more preferably a linear C10-C18 alkyl group.

Examples of OA in the formula (2) include oxyethylene, oxypropylene, and oxybutylene groups.

From the viewpoint of spreadability, the OA is preferably an oxyethylene group.

In the formula (2), m is preferably a number from 3 to 40 from the viewpoint of spreadability.

From the viewpoint of spreadability, the HLB value of the adduct of alkylene oxide with aliphatic alcohol (B) is preferably 8.5 to 11.0, more preferably 9.0 to 10.5.

From the viewpoints of spreadability and persistence of the effect, the weight ratio of polydimethylsiloxane (A) to adduct of alkylene oxide with aliphatic alcohol (B) {(A)/(B)} is preferably 85/15 to 40/60, more preferably 80/20 to 35/65.

From the viewpoint of the persistence of the effect, the total amount of the polydimethylsiloxane (A) and the adduct of alkylene oxide with aliphatic alcohol (B) is preferably 0.1 to 85% by weight, more preferably 1 to 85% by weight, still more preferably 5 to 85% by weight, particularly preferably 40 to 85% by weight, most preferably 50 to 80% by weight, based on the weight of the microbiota improving agent.

From the viewpoint of the persistence of the effect, the total amount of the polydimethylsiloxane (A) and the adduct of alkylene oxide with aliphatic alcohol (B) is preferably 30 to 99% by weight, more preferably 50 to 99% by weight, particularly preferably 80 to 99% by weight, most preferably 90 to 99% by weight, based on the total weight of the substance (X) and the non-volatile component in the base (Y).

When the microbiota improving agent contains only the four components of the substance (X), the polydimethylsiloxane (A), the adduct of alkylene oxide with aliphatic alcohol (B), and water, with the three components other than the substance (X) serving as the base (Y), the total amount of the polydimethylsiloxane (A) and the adduct of alkylene oxide with aliphatic alcohol (B) is preferably 1 to 85% by weight, more preferably 5 to 85% by weight, particularly preferably 40 to 85% by weight, most preferably 50 to 80% by weight, based on the weight of the base (Y), from the viewpoint of the persistence of the effect.

An example of a method for producing the base (Y) containing the polydimethylsiloxane (A), the adduct of alkylene oxide with aliphatic alcohol (B), and water is a method including a step (I) of mixing the polydimethylsiloxane (A), the adduct of alkylene oxide with aliphatic alcohol (B), and water.

Preferably, the polydimethylsiloxane (A) and the adduct of alkylene oxide with aliphatic alcohol (B) are mixed in advance. The mixing of the polydimethylsiloxane (A) and the adduct of alkylene oxide with aliphatic alcohol (B) is preferably performed at a temperature of 20°C to 70°C, more preferably 25°C to 65°C, from the viewpoint of emulsifiability.

In the step (I), the weight ratio of polydimethylsiloxane (A) to adduct of alkylene oxide with aliphatic alcohol (B) {(A)/(B)} is preferably 85/15 to 40/60, more preferably 80/20 to 35/65, from the viewpoints of spreadability and persistence of the effect.

In the step (I), the temperature of the water is preferably 20°C to 70°C, more preferably 25°C to 65°C, from the viewpoint of emulsifiability.

In the step (I), the amount of water is preferably 15 to 99% by weight, more preferably 20 to 99% by weight, based on the total weight of the polydimethylsiloxane (A), the adduct of alkylene oxide with aliphatic alcohol (B), and water, from the viewpoint of the persistence of the effect.

In the step (I), the mixing is preferably performed by stirring with a planetary mixer, from the viewpoint of gelation.

From the viewpoint of uniformity, the rotation speed of the planetary mixer is preferably 10 to 70 rpm for rotation and 5 to 40 rpm for revolution, more preferably 15 to 60 rpm for rotation and 10 to 35 rpm for revolution.

Examples of the stirring blade include a screw blade and a gate blade. From the viewpoint of uniformity, a gate blade is preferred.

In the step (I), when a mixture of the polydimethylsiloxane (A) and the adduct of alkylene oxide with aliphatic alcohol (B) is mixed with water, the water is preferably added in 5 to 50 portions, more preferably in 10 to 40 portions, from the viewpoint of gelation.

Also, in the step (I), from the viewpoint of gelation, water is preferably mixed as follows: a portion of water is added, and the appearance is confirmed to be sufficiently uniform, followed by adding another portion of water.

When the microbiota improving agent of the present invention is used for improving the oral microbiota balance, from the viewpoint of the persistence of the effect, the base (Y) preferably contains (Y-A) and/or (Y-B) as a non-volatile component, which are described later, and more preferably contains both the (Y-A) and the (Y-B).

<(Y-A)>

The (Y-A) is a polymer having an anionic group such as a carboxy group or a carboxylate group (e.g., sodium carboxymethylcellulose, xanthan gum, sodium alginate) and having a weight average molecular weight of 50,000 to 3,000,000, preferably 100,000 to 2,000,000. The (Y-A) may be a mixture of two or more of such polymers.

<(Y-B)>

The (Y-B) is a component having an HLB value of 7 or more excluding the (Y-A) and satisfies the following conditions (1) and/or (2):

(1) a solution having a concentration of 1% by weight of the (Y-B) prepared by dilution with water has a viscosity of 1 mPa·s or higher and lower than 500 mPa·s, preferably 5 mPa·s or higher and 450 mPa·s or lower (the viscosity is measured in the same manner as the viscosity at 37°C of the microbiota improving agent measured with a cone-and-plate rheometer, which is described later); and
(2) the weight average molecular weight is 10,000 to 80,000, preferably 15,000 to 50,000.

The (Y-B) may be a mixture of two or more of the above components.
When the (Y-A) and (Y-B) are contained, the ratio of the weight of the (Y-A) to the weight of the (Y-B) [(Y-A)/(Y-B)] is preferably 0.001 to 0.2, more preferably 0.001 to 0.1.

Other components

The microbiota improving agent of the present invention may contain a component (additive) other than and in addition to the substance (X) and non-volatile components. The additive may be added separately from the base (Y) or may be appropriately contained in the base (Y).
Examples of the additive include lubricants such as wax, antioxidants, ultraviolet absorbers, antifoaming agents, preservatives, fragrances, and solvents such as water and organic solvents (e.g., ethanol).
When the additive contains the following bactericidal component, the total weight of the bactericidal component is preferably 2% by weight or less relative to the total weight of the substance (X), from the viewpoint of the effect of the microbiota improving agent.
The bactericidal component includes at least one selected from the group consisting of glycyrrhizinic acid and salts thereof, β-glycyrrhetinic acid, isopropylmethylphenol, cetylpyridinium chloride, benzalkonium chloride, benzethonium chloride, alkyldiaminoethylglycine chloride solution, chlorhexidine hydrochloride, chlorhexidine hydrochloride, triclosan, lysozyme chloride, hinokitiol, sodium lauroyl sarcosinate, and sodium lauryl sulfate.
The amount of the solvent in the microbiota improving agent is not limited as long as it allows the viscosity and the worked penetration of the microbiota improving agent to fall within the ranges described below. The amount is preferably, for example, 5 to 99% by weight based on the total weight of the microbiota improving agent.
The amount of water in the microbiota improving agent is preferably 5 to 99% by weight, more preferably 30 to 99% by weight, based on the total weight of the microbiota improving agent, from the viewpoints of spreadability and a microbiota balance improving effect.
In the case of the microbiota improving agent containing water, the microbiota improving agent produced by the method described below and allowed to stand at 25°C for 10 minutes preferably has a visually uniform appearance (the agent is not separated into two or more phases).
The upper limit of the amount of additives other than the solvent in the microbiota improving agent is preferably 10% by weight or less, more preferably 8% by weight or less, particularly preferably 1% by weight or less, based on the weight of non-volatile component(s) in the microbiota improving agent.
The lower limit of the amount of additives other than the solvent in the microbiota improving agent is, for example, 0.1% by weight or more based on the weight of non-volatile component(s) in the microbiota improving agent, but is not limited thereto.

Microbiota improving agent

The amount (weight percentage) of the substance (X) is preferably 0.01 to 5% by weight, more preferably 0.05 to 3% by weight, based on the weight of the microbiota improving agent.
From the viewpoint of suitably imparting a high pathogenic bacteria inhibitory effect, the lower limit of the amount (weight percentage) of the substance (X) is preferably 0.01% by weight, more preferably 0.05% by weight, based on the weight of the microbiota improving agent of the present invention.
From the viewpoint of suitably maintaining resident non-pathogenic bacteria, the upper limit of the amount of the substance (X) is preferably 5% by weight, more preferably 3% by weight, based on the weight of the microbiota improving agent of the present invention.
When a hydroxycarboxylic acid (a compound having a hydroxy group and a carboxy group, particularly citric acid) is used as the compound (X2), the weight percentage of the hydroxycarboxylic acid as the compound (X2) is preferably 1% by weight or more based on the weight of the microbiota improving agent, from the viewpoint of the effect of the microbiota

improving agent.

The lower limit of the amount of the substance (X) is preferably 1% by weight, more preferably 2% by weight, relative to the weight of non-volatile component(s) in the microbiota improving agent. The upper limit of the amount of the substance (X) is preferably 200% by weight, more preferably 150% by weight, relative to the weight of non-volatile component(s) in the microbiota improving agent. The amount of the substance (X) is preferably 1 to 200% by weight, more preferably 2 to 150% by weight, relative to the weight of non-volatile component(s) in the microbiota improving agent.

The microbiota improving agent containing the substance (X) in an amount within the above ranges has a high pathogenic bacteria inhibitory effect while maintaining resident non-pathogenic bacteria, and can suitably impart the effect of maintaining the above-described performances for a long period of time.

In the case where the microbiota improving agent of the present invention contains zinc atoms and/or magnesium atoms (including the case where the agent contains a compound containing any of these atoms such as zinc oxide), the total weight percentage of zinc atoms and magnesium atoms is preferably 0.5% by weight or less relative to the total weight of the substance (X), from the viewpoint of the effect of the microbiota improving agent.

In the case where the microbiota improving agent of the present invention contains zinc atoms and/or magnesium atoms (including the case where the agent contains a compound containing any of these atoms), the total weight percentage of zinc atoms and magnesium atoms is preferably 0.005% by weight or less based on the weight of the microbiota improving agent, from the viewpoint of the effect of the microbiota improving agent.

The microbiota improving agent of the present invention has a worked penetration of 100 to 400 as measured in accordance with JIS K 2220. If the worked penetration is less than 100, the pathogenic bacteria inhibitory effect is poor. If the worked penetration exceeds 400, resident non-pathogenic bacteria are not maintained easily.

The worked penetration of the microbiota improving agent of the present invention, measured in accordance with JIS K 2220, is preferably 100 to 300, from the viewpoint of the persistence of the effect.

The viscosity at 37°C of the microbiota improving agent of the present invention, measured with a cone-and-plate rheometer, is 1 to 4,000 Pa·s.

Specifically, the viscosity is measured by the following measurement method.

<Measurement conditions>

- Device: MCR302 (Anton Paar)
- Jig: CP-25 (cone-and-plate diameter: 25 mm)
- Gap: 1 mm
- Temperature: 37°C
- Strain rate: 0.5 (1/s) (rotation speed: 0.6 rpm)

<Measurement method>

Approximately 1.5 g of a sample is placed on the sample stage and sandwiched between the cone and the plate of CP-25, with the gap therebetween being set to 1 mm. The excess sample from the cone and plate is scraped off.

The temperature is adjusted to and maintained at 37°C for one minute, and measurements are performed while changing the rotation speed from 0.1 to 10 rpm (20 plots at measurement intervals of 10 seconds to 1 second). The viscosity value when the strain rate is 0.5 (1/s) (rotation speed: 0.6 rpm) is recorded and taken as the viscosity of the microbiota improving agent.

If the viscosity of the microbiota improving agent is lower than 1 Pa·s, the persistence of the effect of the microbiota improving agent is poor. If the viscosity of the microbiota improving agent is higher than 4,000 Pa·s, the pathogenic bacteria inhibitory effect is poor.

From the viewpoint of the persistence of the effect, the viscosity of the microbiota improving agent is preferably 10 to 4,000 Pa·s, more preferably 1,000 to 4,000 Pa·s.

From the viewpoint of spreadability, the viscosity of the microbiota improving agent is preferably 2,000 to 3,700 Pa·s.

A solution having a concentration of 1% by weight of the microbiota improving agent of the present invention prepared by diluting the microbiota improving agent with water (e.g., ion-exchanged water) preferably has a pH of 6 to 8, from the viewpoint of the microbiota balance improving effect.

The pH can be measured using a pH meter at 25°C.

The microbiota improving agent of the present invention can be produced by adding the components and uniformly mixing them at room temperature or, if necessary, while heating (e.g., 30°C to 70°C). The components may be added in any order and by any method.

Method of use

The microbiota improving agent of the present invention is a microbiota improving agent for improving at least one of mucosal microbiota, skin microbiota, or nail microbiota. The microbiota improving agent can be used as follows: it is applied to the affected area [e.g., mucosa such as oral cavity mucosa (tongue, gums, gingiva, etc.), intestinal mucosa, or eye mucosa, skin, nails (nail plates)] or the vicinity of the affected area with a finger or the like and is left at the affected area. In particular, it is preferably applied to mucosa.

For example, when the microbiota improving agent of the present invention is applied to the oral cavity mucosa (tongue, gums, gingiva, etc.), it is expected to be effective in preventing periodontal disease, which progresses through the stages of gingivitis and periodontitis.

Use of non-volatile components appropriate to the application allows the microbiota improving agent of the present invention to be used as pharmaceuticals, medical devices (medical adhesives, wound healing materials, etc.), quasi-drugs, cosmetics, foods, etc. Use of the agent for these applications can achieve improvement of the microbiota balance in the applied area. Specific examples of the applications include toothpastes, oral gels, oral care foods, supplements, pet care products, and nail care products.

The present specification discloses the following.

The present disclosure (1) relates to a microbiota improving agent for improving at least one of mucosal microbiota, skin microbiota, or nail microbiota, the microbiota improving agent containing:

a substance (X); and
a base (Y) containing a non-volatile component,
the substance (X) including at least one selected from the group consisting of
a compound (X1) having two or more hydroxy groups contained in at least one oxoacid group and a salt of the compound (X1),
a compound (X2) having one hydroxy group contained in an oxoacid group and a salt of the compound (X2),
a heterocyclic compound (X3) having no oxoacid group and a salt of the compound (X3), and
a ketone (X4) having no oxoacid group,
wherein in the compound (X1), a total mass percentage of the at least one oxoacid group, a hydroxy group not contained in the oxoacid group, a carbonyl group not contained in the oxoacid group, carbon atoms contained in an imidazole skeleton and a pyridine skeleton, and nitrogen atoms contained in the imidazole skeleton and the pyridine skeleton is 45% by mass or more based on a molecular weight of the compound (X1),
the compound (X2) has an atom having an unshared electron pair, the atom excluding atoms contained in the oxoacid group and atoms contained in an amino group not constituting an imidazole skeleton and in an amino group not constituting a pyridine skeleton,
in the compound (X2), a total mass percentage of the oxoacid group, a hydroxy group not contained in the oxoacid group, a carbonyl group not contained in the oxoacid group, carbon atoms contained in an imidazole skeleton and a pyridine skeleton, and nitrogen atoms contained in the imidazole skeleton and the pyridine skeleton is 55% by mass or more based on a molecular weight of the compound (X2),
the heterocyclic compound (X3) has an imidazole skeleton or a pyridine skeleton and at least one of a hydroxy group or a carbonyl group, or has two or more skeletons selected from the group consisting of an imidazole skeleton and a pyridine skeleton,
the ketone (X4) has a linear $\beta$-diketone structure,
the substance (X) has a molecular weight of 550 or less,
the non-volatile component has an HLB value of 7 or more, and
the microbiota improving agent satisfies the following conditions (i) and (ii):

(i) the microbiota improving agent has a worked penetration of 100 to 400 as measured in accordance with JIS K 2220; and
(ii) the microbiota improving agent has a viscosity at 37°C of 1 to 4,000 Pa·s as measured with a cone-and-plate rheometer.

The present disclosure (2) relates to the microbiota improving agent according to the present disclosure (1), wherein a solution having a concentration of 1% by weight of the microbiota improving agent prepared by diluting the microbiota improving agent with water has a pH of 6 to 8.

The present disclosure (3) relates to the microbiota improving agent according to the present disclosure (1) or (2), wherein a weight percentage of the substance (X) is 0.01 to 5% by weight based on a weight of the microbiota improving agent.

EXAMPLES

The present invention will be further described below with reference to examples and comparative examples, but the present invention is not limited thereto. In the following description, unless otherwise specified, "part(s)" means "part(s) by weight and "%" means "% by weight".

<Examples 1 to 44 and Comparative Examples 1 to 16: Production of microbiota improving agent>

A substance (X) or (X'), a base (Y) or (Y'), and ion-exchanged water were mixed at 25°C to obtain microbiota improving agents having the corresponding concentrations (% by weight) of these components shown in Tables 1-1 to 1-6.

The microbiota improving agents were subjected to measurements of viscosity and worked penetration by the methods described herein. The results are shown in Tables 1-1 to 1-6. Solutions having a concentration of 1% by weight of the microbiota improving agents were prepared by diluting the microbiota improving agents with water. The pHs of the solutions are also shown in Tables 1-1 to 1-6. The worked penetration of the microbiota improving agent of Comparative Example 14 exceeded 400 and was impossible to measure.

Solutions having a concentration of 1% by weight of the non-volatile components in the bases (Y) were prepared by dilution with ion-exchanged water. The viscosities of the solutions measured by the method described herein, and the HLB values and weight average molecular weights of the non-volatile components are shown in Table 2.

<Description of components>

The following describes the raw materials corresponding to the components used in the examples and comparative examples.

Substance (X) or (X')

(X-1): Pentasodium triphosphate (FUJIFILM Wako Pure Chemical Corporation, the total mass percentage of the oxoacid groups based on the molecular weight of triphosphoric acid: 88% by mass)
(X-2): Acetylacetone (Nacalai Tesque, Inc., the total mass percentage of the carbonyl groups based on the molecular weight of acetylacetone: 56% by mass)
(X-3): Benzoylacetone (MERCK, the total mass percentage of the carbonyl groups based on the molecular weight of benzoylacetone: 35% by mass)
(X-4): Levulinic acid (Tokyo Chemical Industry Co., Ltd., the total mass percentage of the oxoacid group and carbonyl group based on the molecular weight of levulinic acid: 63% by mass)
(X-5): Sodium tungstate dihydrate ($Na_2WO_4 \cdot 2H_2O$ available from FUJIFILM Wako Pure Chemical Corporation, the total mass percentage of the oxoacid group based on the molecular weight of tungstic acid: 100% by mass); the weight percentage of the substance (X) ($Na_2WO_4$) contained in (X-5) is 89% by weight based on the weight of (X-5)
(X-6): Glycylglycine (FUJIFILM Wako Pure Chemical Corporation, the total mass percentage of the oxoacid group and carbonyl group based on the molecular weight of glycylglycine: 55% by mass)
(X-7): Glutamic acid (FUJIFILM Wako Pure Chemical Corporation, the total mass percentage of the oxoacid groups based on the molecular weight of glutamic acid: 61% by mass)
(X-8): Malic acid (FUJIFILM Wako Pure Chemical Corporation, the total mass percentage of the oxoacid groups and hydroxy group based on the molecular weight of malic acid: 80% by mass)
(X-9): Citric acid (FUJIFILM Wako Pure Chemical Corporation, the total mass percentage of the oxoacid groups and hydroxy group based on the molecular weight of citric acid: 79% by mass)
(X-10): Gluconic acid (FUJIFILM Wako Pure Chemical Corporation, the total mass percentage of the oxoacid group and hydroxy groups based on the molecular weight of gluconic acid: 66% by mass)
(X-11): Glucuronic acid (FUJIFILM Wako Pure Chemical Corporation, the total mass percentage of the oxoacid group and hydroxy groups based on the molecular weight of glucuronic acid: 58% by mass]
(X-12): Ethylenediaminetetraacetic acid (FUJIFILM Wako Pure Chemical Corporation, the total mass percentage of the oxoacid groups based on the molecular weight of ethylenediaminetetraacetic acid: 62% by mass)
(X-13): Iminodiacetic acid (FUJIFILM Wako Pure Chemical Corporation, the total mass percentage of the oxoacid groups based on the molecular weight of iminodiacetic acid: 68% by mass)
(X-14): 8-Quinolinol (FUJIFILM Wako Pure Chemical Corporation, the total mass percentage of the carbon atoms and the nitrogen atom contained in the pyridine skeleton and the hydroxy group based on the molecular weight of 8-quinolinol: 63% by mass)

(X-15): Tartaric acid (FUJIFILM Wako Pure Chemical Corporation, the total mass percentage of the oxoacid groups and hydroxy groups based on the molecular weight of tartaric acid: 83% by mass)
(X-16): Adenosine triphosphate disodium trihydrate (FUJIFILM Wako Pure Chemical Corporation, the total mass percentage of the oxoacid groups, the hydroxy groups, and the carbon atoms and nitrogen atoms contained in the imidazole skeleton based on the molecular weight of adenosine triphosphate: 61% by mass); the weight percentage of the substance (X) (adenosine triphosphate disodium) contained in (X-16) is 91% by weight based on the weight of (X-16)
(X'-1): Benzalkonium chloride (FUJIFILM Wako Pure Chemical Corporation)
(X'-2): Cetylpyridinium chloride (FUJIFILM Wako Pure Chemical Corporation)

Base (Y) or (Y')

(Y-1): Carboxymethylethylcellulose (product name: CMEC, available from Sanyo Chemical Industries, Ltd.)
(Y-2): Sodium carboxymethylcellulose (FUJIFILM Wako Pure Chemical Corporation)
(Y-3): Sodium alginate (FUJIFILM Wako Pure Chemical Corporation)
(Y-4): Xanthan gum (FUJIFILM Wako Pure Chemical Corporation)
(Y-5): Starch-sodium acrylate graft copolymer (product name: Sunfresh ST100, available from Sanyo Chemical Industries, Ltd.)
(Y-6): PEG 20000 (Sanyo Chemical Industries, Ltd.)
(Y-7-1) to (Y-7-4): produced in Production Examples 1 to 4, respectively
(Y'-1): White petrolatum (FUJIFILM Wako Pure Chemical Corporation)
(Y-8): Methylcellulose 4000 (FUJIFILM Wako Pure Chemical Corporation)

Each substance (X) was subjected to measurement of the amount (g) of magnesium captured per 100 g of the substance (X) by the ion-selective electrode method as follows. For (X-5), the amount of magnesium captured per 100 g of $Na_2WO_4$ contained in (X-5) was measured. For (X-16), the amount of magnesium captured per 100 g of adenosine triphosphate disodium contained in (X-16) was measured.
The measurement by the ion-selective electrode method was performed using 2 mM of the substance (X) relative to a 1 mM aqueous $MgCl_2$ solution having a temperature of 20°C and a pH of 7.
The results are shown in Table 3.

<Production Example 1: Production of base (Y-7-1)>

A planetary mixer PLM-15 equipped with a gate blade (Inoue Mfg., Inc.) was charged with 46.9 parts of polydimethylsiloxane (Dow Corning Toray Co., Ltd., Q7-9120 Silicone Fluid 1000 cSt (n = 350), HLB value: 5.1) and 23.1 parts of an adduct of oxyethylene (15 mol) with cetyl alcohol (HLB value: 12.2). The contents were heated to 37°C, and then the temperature was maintained at 37°C while the contents were stirred in the planetary mixer at a rotation speed of 20 rpm and a revolution speed of 50 rpm. To the mixer was gradually added 40 parts of warm water adjusted to 37°C while checking the appearance. Thereby, a base (Y-7-1) was prepared.

<Production Example 2: Production of base (Y-7-2)>

A base (Y-7-2) was prepared as in Production Example 1, except that "630 parts of warm water" was used instead of "40 parts of warm water".

<Production Example 3: Production of base (Y-7-3)>

A base (Y-7-3) was prepared as in Production Example 1, except that "3,430 parts of warm water" was used instead of "40 parts of warm water".

<Production Example 4: Production of base (Y-7-4)>

A base (Y-7-4) was prepared as in Production Example 1, except that "20 parts of warm water" was used instead of "40 parts of warm water".

<Confirmation test for selective inhibition of bacterial growth>

The pathogenic bacteria used in the test were *Porphyromonas gingivalis, Fusobacterium nucleatum, Prevotella intermedia, Trichophyton rubrum, Trichophyton mentagrophytes, Staphylococcus aureus, Candida albicans,* and *Candida glabrata,* while the non-pathogenic bacteria used were *Streptococcus salivarius* and *Staphylococcus epidermidis.*

These bacteria were cultured using the following media. A medium prepared by adding 1 ug/mL menadione to modified GAM Broth "Nissui" (Nissui Pharmaceutical Co., Ltd.) for *Porphyromonas gingivalis;* modified GAM Broth "Nissui" was used for *Fusobacterium nucleatum, Prevotella intermedia,* and *Streptococcus salivarius;* Sabouraud-dextrose broth (Nippon Pharmaceutical Co., Ltd.) was used for *Trichophyton rubrum* and *Trichophyton mentagrophytes;* LB medium (Becton Dickinson) was used for *Staphylococcus aureus* and *Staphylococcus epidermidis;* and YM medium (Sigma) was used for *Candida albicans* and *Candida glabrata.*

*Porphyromonas gingivalis, Fusobacterium nucleatum,* and *Prevotella intermedia* were cultured under anaerobic conditions, while *Streptococcus salivarius, Trichophyton rubrum, Trichophyton mentagrophytes, Staphylococcus aureus, Staphylococcus epidermidis, Candida albicans,* and *Candida glabrata* were cultured under aerobic conditions.

Regarding the microbiota improving agents prepared according to the formulations shown in Tables 1-1 to 1-6, 50 mg of any of the microbiota improving agents was added into each well of 24-well plates.

Separately, the bacterial strains were cultured in the corresponding media overnight at 37°C under anaerobic conditions and diluted with the same culture media so that the dilutions had an absorbance at 600 nm of 0.2. Then, 500 μL of any of the dilutions was added into each well of a 24-well plate containing the microbiota improving agent, and static culture was performed at 37°C under anaerobic conditions using AnaeroPack-Anaero 10% (Mitsubishi Gas Chemical Company, Inc.). Separately, the bacterial strains were cultured in the corresponding media overnight with shaking at 37°C under aerobic conditions and diluted with the same culture media so that the dilutions had an absorbance at 600 nm of 0.2. Then, 500 μL of any of the dilutions was added into each well of a 24-well plate containing the microbiota improving agent, and static culture was performed at 37°C. After 48 hours, each plate was collected and the absorbance at 600 nm was measured to evaluate the growth characteristics of the bacteria.

The growth inhibitory effect was expressed as a relative value to the turbidity, which was taken as 1, in the case of using the same weight of ion-exchanged water instead of the microbiota improving agent. The results are shown in Tables 1-1 to 1-6.

In the case of applying the microbiota improving agent of the present invention to the oral cavity, when the growth characteristics of *Porphyromonas gingivalis, Fusobacterium nucleatum,* and *Prevotella intermedia* are more inhibited with the microbiota improving agent than without it, and the growth characteristics of *Streptococcus salivarius* remain unchanged from those without the microbiota improving agent, the bacterial growth is considered to be inhibited selectively.

For example, the relative values may fall within the following ranges.

The results of the tests for *Porphyromonas gingivalis, Fusobacterium nucleatum,* and *Prevotella intermedia* show that each of the relative values is 0.01 to 0.30, preferably 0.01 to 0.15, and the result of the test for *Streptococcus salivarius* shows that the relative value is 0.8 to 1.2.

In the case of applying the microbiota improving agent of the present invention to the skin and nail plates, when the growth characteristics of *Trichophyton rubrum, Trichophyton mentagrophytes, Staphylococcus aureus, Candida albicans,* and *Candida glabrata* are more inhibited with the microbiota improving agent than without it, and the growth characteristics of *Staphylococcus epidermidis* remain unchanged from those without the microbiota improving agent, the bacterial growth is considered to be inhibited selectively.

For example, the relative values may fall within the following ranges.

The results of the tests for *Trichophyton rubrum, Trichophyton mentagrophytes,* and *Staphylococcus aureus* show that each of the relative values are 0.01 to 0.30, preferably 0.01 to 0.15, and the result of the test for *Staphylococcus epidermidis* shows that the relative value is 0.8 to 1.2.

<Assay of inhibition of protease production by pathogenic bacteria>

The pathogenic bacteria used in the assay were *Porphyromonas gingivalis, Fusobacterium nucleatum,* and *Prevotella intermedia.* These bacteria were cultured using the following media. A medium prepared by adding 1 μg/mL menadione to modified GAM broth "Nissui" (Nissui Pharmaceutical Co., Ltd.) was used for *Porphyromonas gingivalis,* and modified GAM broth "Nissui" was used for *Fusobacterium nucleatum* and *Prevotella intermedia.*

Regarding the microbiota improving agents prepared according to the formulations shown in Tables 1-1 to 1-6, 50 mg of any of the microbiota improving agents was added into each well of a 24-well plate. Separately, the bacterial strains were cultured in the corresponding media overnight at 37°C under anaerobic conditions and diluted with the same culture media so that the dilutions had an absorbance at 600 nm of 0.2. Then, 500 μL of any of the dilutions was added into each well of the 24-well plate containing the microbiota improving agent, and static culture was performed at 37°C under

anaerobic conditions using AnaeroPack-Anaero 10% (Mitsubishi Gas Chemical Company, Inc.). After 48 hours, the plate was collected and the culture was centrifuged at 4°C and 10,000 rpm for five minutes to collect the culture supernatant. The activity of protease contained in the culture supernatant was evaluated using an Amplite protease activity assay kit (AAT Bioquest).

The protease production was expressed as a relative value to the protease activity value, which was taken as 1, in the case of using the same weight of ion-exchanged water instead of the microbiota improving agent. The results are shown in Tables 1-1 to 1-6.

Protease is known to be one of the toxin components produced by pathogenic bacteria. The lower the protease activity value, the more inhibited the protease production.

<Evaluation of persistence of effect by halo test>

Preparation of agar medium for halo test

The pathogenic bacteria used in the test were *Porphyromonas gingivalis, Fusobacterium nucleatum, Prevotella intermedia, Trichophyton rubrum, Trichophyton mentagrophytes, Staphylococcus aureus, Candida albicans,* and *Candida glabrata,* while the non-pathogenic bacteria used were *Streptococcus salivarius* and *Staphylococcus epidermidis.*

These bacteria were cultured using the following media. A medium prepared by adding 1 ug/mL menadione to modified GAM Broth "Nissui" (Nissui Pharmaceutical Co., Ltd.) was used for *Porphyromonas gingivalis;* modified GAM broth "Nissui" was used for *Fusobacterium nucleatum, Prevotella intermedia,* and *Streptococcus salivarius;* Sabouraud-dextrose broth (Nippon Pharmaceutical Co., Ltd.) was used for *Trichophyton rubrum* and *Trichophyton mentagrophytes;* LB medium (Becton Dickinson) was used for *Staphylococcus aureus* and *Staphylococcus epidermidis;* and YM medium (Sigma) was used for *Candida albicans* and *Candida glabrata.*

The bacterial strains were cultured in the corresponding media overnight at 37°C under anaerobic or aerobic conditions and made into bacterial solutions of $1.0 \times 10^5$ CFU/ml. Then, 100 μL of any of the bacterial solutions was inoculated on an agar medium prepared from any of the media and uniformly spread with a bacteria spreader.

Halo test

Regarding the microbiota improving agents prepared according to the formulations shown in Tables 1-1 to 1-6, 200 μL of any of the microbiota improving agents as a test sample was inoculated on the agar medium inoculated with any of the bacteria, followed by testing. The culture of bacteria under anaerobic conditions was performed at 37°C for 24 hours using AnaeroPack-Anaero 10% (Mitsubishi Gas Chemical Company, Inc.). The culture of bacteria under aerobic conditions was performed at 37°C for 24 hours. In the evaluation of the persistence of the growth inhibitory effect on pathogenic bacteria, for the agents rated as × (Poor) after 24 hours of culture, the culture of pathogenic bacteria was stopped, whereas for the other agents, the culture was continued at 37°C for one week. For the agents rated as Δ (Fair) after one week of culture, the culture of pathogenic bacteria was stopped, whereas for the other agents, the culture was continued at 37°C for two weeks. In the evaluation of the persistence of the effect on non-pathogenic bacteria, all the bacteria were cultured for two weeks.

The presence of a halo was determined by measuring the length from the edge of the test sample application area to the edge of a halo. When the length was greater than 0 mm, a halo was determined to be present, whereas when the length was 0 mm, a halo was determined to be absent.

In the assay of the growth inhibitory effect on pathogenic bacteria, the presence of a halo indicates having a growth inhibitory effect, whereas the absence of a halo indicates having no growth inhibitory effect. In the assay of the effect on non-pathogenic bacteria, the absence of a halo indicates no inhibition of the growth (growth is unaffected), whereas the presence of a halo indicates inhibition of the growth. The assay of the persistence of the growth inhibitory effect on pathogenic bacteria (persistence assay (pathogenic bacteria)) and the assay of the persistence of the effect on non-pathogenic bacteria (persistence assay (non-pathogenic bacteria)) were performed according to the following criteria. The evaluation results are shown in Tables 1-1 to 1-6.

<Persistence assay (pathogenic bacteria)>

◦◦ (Excellent): A halo was observed in the assays after 24 hours of culture, one week of culture, and two weeks of culture.

◦ (Good): A halo was observed in the assays after 24 hours of culture and one week of culture, but a halo was not observed (had disappeared) in the assay after two weeks of culture.

∆ (Fair): A halo was observed in the assay after 24 hours of culture, but was not observed in the assay after one week of culture.
× (Poor): No halo was observed in the assay after 24 hours of culture.

<Persistence assay (non-pathogenic bacteria)>

◦◦ (Excellent): A halo was not observed even after 24 hours of culture, one week of culture, and two weeks of culture.
◦ (Good): A halo was observed in the assay after 24 hours of culture, but was not observed in the assays after one week of culture and two weeks of culture.
∆ (Fair): A halo was observed in the assays after 24 hours of culture and one week of culture, but was not observed in the assay after two weeks of culture.
× (Poor): A halo was observed in the assays after 24 hours of culture, one week of culture, and two weeks of culture.

<Evaluation of spreadability>

A 10 cm square piece of leather was immersed in 80 cc of artificial saliva (0.844 g/L sodium chloride, 1.2 g/L potassium chloride, 0.146 g/L calcium chloride hydrate, 0.052 g/L magnesium chloride, 0.342 g/L dipotassium phosphate) contained in a 15 cm diameter petri dish at 37°C for 10 minutes. After 10 minutes, the moisture on the leather surface was removed with gauze. Sensory tests were conducted by 10 panelists as follows: 0.5 g of any of the microbiota improving agents was applied to the piece of leather with a finger; and the smoothness during the application and the amount of the microbiota improving agent remaining on the finger were evaluated. They were evaluated as follows and the results are shown in Tables 1-1 to 1-6.

◦◦ (Excellent): Seven or more panelists felt that the microbiota improving agent was smoothly applied and that no microbiota improving agent remained on their fingers.
◦ (Good): Seven or more panelists felt that the microbiota improving agent was smoothly applied but that the microbiota improving agent remained on their fingers.
∆ (Fair): Seven or more panelists felt that the agent was not smoothly applied and that no microbiota improving agent remained on their fingers.
× (Poor): Seven or more panelists felt that the microbiota improving agent was not smoothly applied and that the microbiota improving agent remained on their fingers.

[Table 1-1]

| | | Example | | | | | | | | | |
|---|---|---|---|---|---|---|---|---|---|---|---|
| | | 1 | 2 | 3 | 4 | 5 | 6 | 7 | 8 | 9 | 10 |
| Substance (X) or (X') | X-1 | 0.05 | 1.0 | 3.0 | 3.0 | 3.0 | 3.0 | 3.0 | - | - | - |
| | X-2 | - | - | - | - | - | - | - | 3.0 | - | - |
| | X-3 | - | - | - | - | - | - | - | - | 3.0 | - |
| | X-4 | - | - | - | - | - | - | - | - | - | 3.0 |
| | X-5 | - | - | - | - | - | - | - | - | - | - |
| | X-6 | - | - | - | - | - | - | - | - | - | - |
| | X-7 | - | - | - | - | - | - | - | - | - | - |
| | X-8 | - | - | - | - | - | - | - | - | - | - |
| | X-9 | - | - | - | - | - | - | 0.35 | - | - | - |
| | X-10 | - | - | - | - | - | - | - | - | - | - |
| | X-11 | - | - | - | - | - | - | - | - | - | - |
| | X-12 | - | - | - | - | - | - | - | - | - | - |
| | X-13 | - | - | - | - | - | - | - | - | - | - |
| | X-14 | - | - | - | - | - | - | - | - | - | - |
| | X-15 | - | - | - | - | - | - | - | - | - | - |
| | X-16 | - | - | - | - | - | - | - | - | - | - |
| | X'-1 | - | - | - | - | - | - | - | - | - | |
| | X'-2 | - | - | - | - | - | - | - | - | - | - |
| Base (Y) or (Y') | Y-1 | 2.0 | 2.0 | 2.0 | - | - | - | - | - | - | - |
| | Y-2 | - | - | - | 2.0 | - | - | - | - | - | - |
| | Y-3 | - | - | - | - | - | - | - | - | - | - |
| | Y-4 | - | - | - | - | 5.0 | 5.0 | 5.0 | 5.0 | 5.0 | 5.0 |
| | Y-5 | - | - | - | - | - | - | - | - | - | - |
| | Y-6 | - | - | - | - | - | - | - | - | - | - |
| | Y-7-1 | - | - | - | - | - | - | - | - | - | - |

(continued)

| | | | Example | | | | | | | | | |
|---|---|---|---|---|---|---|---|---|---|---|---|---|
| | | | 1 | 2 | 3 | 4 | 5 | 6 | 7 | 8 | 9 | 10 |
| | | Y-7-2 | - | - | - | - | - | - | - | - | - | - |
| | | Y-7-3 | - | - | - | - | - | - | - | - | - | - |
| | | Y'-1 | - | - | - | - | - | - | - | - | - | - |
| | | Y-7-4 | - | - | - | - | - | - | - | - | - | - |
| | | Y-8 | - | - | - | - | - | 10.0 | - | - | - | - |
| Volatile component | | Ion-exchanged water | 97.95 | 97.00 | 95.00 | 95.00 | 92.00 | 82.00 | 91.65 | 92.00 | 92.00 | 92.00 |
| Viscosity of microbiota improving agent (Pa·s) | | | 5.5 | 5.5 | 5.5 | 13.1 | 18.1 | 20 | 18.1 | 18.2 | 17.9 | 18.1 |
| Worked penetration of microbiota improving agent | | | 290 | 286 | 292 | 279 | 280 | 250 | 280 | 275 | 279 | 280 |
| pH of solution having concentration of 1% by weight of microbiota improving agent prepared by dilution with water | | | 7.2 | 7.1 | 6.9 | 7.0 | 7.2 | 7.2 | 7.2 | 7,1 | 7.2 | 6.9 |
| Confirmation test for selective inhibition of bacterial growth | Pathogenic bacteria | *Porphyromonas gingivalis* | 0.1 | 0.07 | 0.04 | 0.05 | 0.05 | 0.05 | 0.05 | 0.06 | 0.05 | 0.05 |
| | | *Fusobacterium nucleatum* | 0.11 | 0.08 | 0.04 | 0.05 | 0.05 | 0.05 | 0.05 | 0.05 | 0.05 | 0.05 |
| | | *Prevotella intermedia* | 0.09 | 0.07 | 0.05 | 0.05 | 0.05 | 0.05 | 0.05 | 0.05 | 0.06 | 0.05 |
| | | *Trichophyton rubrum* | 0.08 | 0.07 | 0.05 | 0.04 | 0.05 | 0.05 | 0.05 | 0.05 | 0.06 | 0.05 |
| | | *Trichophyton* | 0.09 | 0.07 | 0.05 | 0.05 | 0.05 | 0.05 | 0.05 | 0.05 | 0.07 | 0.05 |
| | | *Staohylococcus aureus* | 0.09 | 0.07 | 0.05 | 0.05 | 0.05 | 0.05 | 0.05 | 0.05 | 0.06 | 0.05 |
| | | *Candida albicans* | 0.2 | 0.09 | 0.05 | 0.05 | 0.05 | 0.05 | 0.05 | 0.05 | 0.05 | 0.05 |
| | | *Candida alabrata* | 0.18 | 0.06 | 0.05 | 0.05 | 0.05 | 0.05 | 0.05 | 0.05 | 0.05 | 0.05 |
| | Non-pathogenic bacteria | *Streptococcus salivarius* | 0.9 | 1.0 | 1.0 | 1.1 | 0.9 | 0.9 | 0.9 | 0.9 | 1.0 | 1.0 |
| | | *Staphylococcus epidermidis* | 1.0 | 1.1 | 0.9 | 1.0 | 1.0 | 0.9 | 1.0 | 0.9 | 1.0 | 0.9 |

(continued)

| | | | Example | | | | | | | | | |
|---|---|---|---|---|---|---|---|---|---|---|---|---|
| | | | 1 | 2 | 3 | 4 | 5 | 6 | 7 | 8 | 9 | 10 |
| Inhibition assay of protease production by pathogenic bacteria | | *Porphyromonas gingivalis* | 0.001 | 0.001 | 0.001 | 0.001 | 0.001 | 0.001 | 0.001 | 0.001 | 0.001 | 0.001 |
| | | *Fusobacterium nucleatum* | 0.002 | 0.001 | 0.001 | 0.001 | 0.001 | 0.001 | 0.001 | 0.001 | 0.002 | 0.001 |
| | | *Prevotella intermedia* | 0.001 | 0.001 | 0.002 | 0.001 | 0.001 | 0.001 | 0.001 | 0.001 | 0.001 | 0.001 |
| Persistence evaluation | Pathogenic bacteria | *Porphyromonas gingivalis* | △ | △ | △ | ○ | ○ | ○○ | ○ | ○ | ○ | ○ |
| | | *Fusobacterium nucleatum* | △ | △ | △ | ○ | ○ | ○○ | ○ | ○ | ○ | ○ |
| | | *Prevotella intermedia* | △ | △ | △ | ○ | ○ | ○○ | ○ | ○ | ○ | ○ |
| | | *Trichophyton rubrum* | △ | △ | △ | ○ | ○ | ○○ | ○ | ○ | ○ | ○ |
| | | *Trichophyton* | △ | △ | △ | ○ | ○ | ○○ | ○ | ○ | ○ | ○ |
| | | *Staohylococcus aureus* | △ | △ | △ | ○ | ○ | ○○ | ○ | ○ | ○ | ○ |
| | | *Candida albicans* | △ | △ | △ | ○ | ○ | ○○ | ○ | ○ | ○ | ○ |
| | | *Candida glabrata* | △ | △ | △ | ○ | ○ | ○○ | ○ | ○ | ○ | ○ |
| | Non-pathogenic bacteria | *Streptococcus salivarius* | ○○ | ○○ | ○○ | ○○ | ○○ | ○○ | ○○ | ○○ | ○○ | ○○ |
| | | *Staphylococcus epidermidis* | ○○ | ○○ | ○○ | ○○ | ○○ | ○○ | ○○ | ○○ | ○○ | ○○ |
| Spreadability evaluation | | | ○ | ○ | ○ | ○ | ○ | ○ | ○ | ○ | ○ | ○ |

[Table 1-2]

| | | Example | | | | | | | | | |
|---|---|---|---|---|---|---|---|---|---|---|---|
| | | 11 | 12 | 13 | 14 | 15 | 16 | 17 | 18 | 19 | 20 |
| Substance (X) or (X') | X-1 | - | - | - | - | - | - | - | - | - | - |
| | X-2 | - | - | - | - | - | - | - | - | - | - |
| | X-3 | - | - | - | - | - | - | - | - | - | - |
| | X-4 | - | - | - | - | - | - | - | - | - | - |
| | X-5 | 3.0 | - | - | - | - | - | - | - | - | - |
| | X-6 | - | 3.0 | - | - | - | - | - | - | - | - |
| | X-7 | - | - | 3.0 | - | - | - | - | - | - | - |
| | X-8 | - | - | - | 3.0 | - | - | - | - | - | - |
| | X-9 | - | - | - | - | 3.0 | - | - | - | - | - |
| | X-10 | - | - | - | - | - | 3.0 | - | - | - | - |
| | X-11 | - | - | - | - | - | - | 3.0 | - | - | - |
| | X-12 | - | - | - | - | - | - | - | 3.0 | - | - |
| | X-13 | - | - | - | - | - | - | - | - | 3.0 | - |
| | X-14 | - | - | - | - | - | - | - | - | - | 3.0 |
| | X-15 | - | - | - | - | - | - | - | - | - | - |
| | X-16 | - | - | - | - | - | - | - | - | - | - |
| | X'-1 | - | - | - | - | - | - | - | - | - | - |
| | X'-2 | - | - | - | - | - | - | - | - | - | - |
| Base (Y) or (Y') | Y-1 | - | - | - | - | - | - | - | - | - | - |
| | Y-2 | - | - | - | - | - | - | - | - | - | - |
| | Y-3 | - | - | - | - | - | - | - | - | - | - |
| | Y-4 | 5.0 | 5.0 | 5.0 | 5.0 | 5.0 | 5.0 | 5.0 | 5.0 | 5.0 | 5.0 |
| | Y-5 | - | - | - | - | - | - | - | - | - | - |
| | Y-6 | - | - | - | - | - | - | - | - | - | - |
| | Y-7-1 | - | - | - | - | - | - | - | - | - | - |

(continued)

| | | | Example | | | | | | | | | |
|---|---|---|---|---|---|---|---|---|---|---|---|---|
| | | | 11 | 12 | 13 | 14 | 15 | 16 | 17 | 18 | 19 | 20 |
| | | Y-7-2 | - | - | - | - | - | - | - | - | - | - |
| | | Y-7-3 | - | - | - | - | - | - | - | - | - | - |
| | | Y'-1 | - | - | - | - | - | - | - | - | - | - |
| | | Y-7-4 | - | - | - | - | - | - | - | - | - | - |
| | | Y-8 | - | - | - | - | - | - | - | - | - | - |
| Volatile component | | Ion-exchanged water | 92.00 | 92.00 | 92.00 | 92.00 | 92.00 | 92.00 | 92.00 | 92.00 | 92.00 | 92.00 |
| Viscosity of microbiota improvina aaent (Pa·s) | | | 17.7 | 18.2 | 18.0 | 17.7 | 17.5 | 18.1 | 18.1 | 17.7 | 17.7 | 17.9 |
| Worked penetration of microbiota improving agent | | | 278 | 275 | 278 | 278 | 277 | 274 | 274 | 278 | 278 | 278 |
| pH of solution having concentration of 1% by weight of microbiota improving agent prepared by dilution with water | | | 7.0 | 7.0 | 6.9 | 7.0 | 6.4 | 6.5 | 7.0 | 7.5 | 7.0 | 7.2 |
| Confirmation test for selective inhibition of bacterial growth | Pathogenic bacteria | *Porphyromonas ainaivalis* | 0.05 | 0.05 | 0.06 | 0.05 | 0.1 | 0.05 | 0.05 | 0.05 | 0.05 | 0.05 |
| | | *Fusobacterium nucleatum* | 0.05 | 0.05 | 0.05 | 0.06 | 0.09 | 0.05 | 0.05 | 0.06 | 0.05 | 0.06 |
| | | *Prevotella intermedia* | 0.06 | 0.06 | 0.06 | 0.06 | 0.08 | 0.06 | 0.06 | 0.06 | 0.05 | 0.06 |
| | | *Trichophyton rubrum* | 0.05 | 0.05 | 0.05 | 0.05 | 0.1 | 0.06 | 0.05 | 0.05 | 0.05 | 0.05 |
| | | *TrichOphyton mentagrophytes* | 0.06 | 0.06 | 0.05 | 0.06 | 0.11 | 0.06 | 0.06 | 0.06 | 0.06 | 0.06 |
| | | *Stapjylococcus aureus* | 0.06 | 0.06 | 0.06 | 0.05 | 0.1 | 0.06 | 0.06 | 0.06 | 0.06 | 0.06 |
| | | *Candida albicans* | 0.05 | 0.05 | 0.05 | 0.07 | 0.2 | 0.05 | 0.05 | 0.05 | 0.06 | 0.05 |
| | | *Candida alabrata* | 0.05 | 0.05 | 0.05 | 0.05 | 0.15 | 0.06 | 0.05 | 0.05 | 0.05 | 0.05 |

(continued)

| | | | Example | | | | | | | | | |
|---|---|---|---|---|---|---|---|---|---|---|---|---|
| | | | 11 | 12 | 13 | 14 | 15 | 16 | 17 | 18 | 19 | 20 |
| | Non-pathogenic bacteria | *Streptococcus salivarius* | 1.1 | 1.1 | 1.0 | 0.9 | 1.1 | 1.1 | 1.0 | 1.1 | 0.9 | 1.1 |
| | | *Staphylococcus epidermidis* | 1.0 | 1.0 | 1.0 | 1.0 | 0.9 | 1.0 | 1.0 | 1.0 | 1.0 | 0.9 |
| Inhibition assay of protease production by pathogenic bacteria | | *Porphyromonas gingivalis* | 0.001 | 0.001 | 0.001 | 0.001 | 0.003 | 0.001 | 0.001 | 0.001 | 0.001 | 0.001 |
| | | *Fusobacterium nucleatum* | 0.002 | 0.002 | 0.002 | 0.002 | 0.002 | 0.002 | 0.002 | 0.002 | 0.002 | 0.002 |
| | | *Prevotella intermedia* | 0.001 | 0.001 | 0.001 | 0.001 | 0.001 | 0.001 | 0.001 | 0.001 | 0.001 | 0.001 |
| Persistence evaluation | Pathogenic bacteria | *Porphyromonas gingivalis* | ○ | ○ | ○ | ○ | △ | ○ | ○ | ○ | ○ | ○ |
| | | *Fusobacterium nucleatum* | ○ | ○ | ○ | ○ | △ | ○ | ○ | ○ | ○ | ○ |
| | | *Prevotella intermedia* | ○ | ○ | ○ | ○ | △ | ○ | ○ | ○ | ○ | ○ |
| | | *Trichophyton rubrum* | ○ | ○ | ○ | ○ | △ | ○ | ○ | ○ | ○ | ○ |
| | | *Trichophyton mentagrophytes* | ○ | ○ | ○ | ○ | △ | ○ | ○ | ○ | ○ | ○ |
| | | *Staphylococcus aureus* | ○ | ○ | ○ | ○ | △ | ○ | ○ | ○ | ○ | ○ |
| | | *Candida albicans* | ○ | ○ | ○ | ○ | △ | ○ | ○ | ○ | ○ | ○ |
| | | *Candida alabrata* | ○ | ○ | ○ | ○ | △ | ○ | ○ | ○ | ○ | ○ |
| | Non-pathogenic bacteria | *Streptococcus salivarius* | ○○ | ○○ | ○○ | ○○ | ○○ | ○○ | ○○ | ○○ | ○○ | ○○ |
| | | *Staphylococcus epidermidis* | ○○ | ○○ | ○○ | ○○ | ○○ | ○○ | ○○ | ○○ | ○○ | ○○ |
| Spreadability evaluation | | | ○ | ○ | ○ | ○ | ○ | ○ | ○ | ○ | ○ | ○ |

[Table 1-3]

| | | Example | | | | | | | | | |
|---|---|---|---|---|---|---|---|---|---|---|---|
| | | 21 | 22 | 23 | 24 | 25 | 26 | 27 | 28 | 29 | 30 |
| Substance (X) or (X') | X-1 | - | - | - | 3.0 | 3.0 | 3.0 | 3.0 | - | - | - |
| | X-2 | - | - | - | - | - | - | - | 3.0 | - | - |
| | X-3 | - | - | - | - | - | - | - | - | 3.0 | - |
| | X-4 | - | - | - | - | - | - | - | - | - | 3.0 |
| | X-5 | - | - | - | - | - | - | - | - | - | - |
| | X-6 | - | - | - | - | - | - | - | - | - | - |
| | X-7 | - | - | - | - | - | - | - | - | - | - |
| | X-8 | - | - | - | - | - | - | - | - | - | - |
| | X-9 | - | - | 1.0 | - | - | - | - | - | - | - |
| | X-10 | - | - | - | - | - | - | - | - | - | - |
| | X-11 | - | - | - | - | - | - | - | - | - | - |
| | X-12 | - | - | - | - | - | - | - | - | - | - |
| | X-13 | - | - | - | - | - | - | - | - | - | - |
| | X-14 | - | - | - | - | - | - | - | - | - | - |
| | X-15 | 3.0 | - | - | - | - | - | - | - | - | - |
| | X-16 | - | 3.0 | - | - | - | - | - | - | - | - |
| | X'-1 | - | - | - | - | - | - | - | - | - | - |
| | X'-2 | - | - | - | 0.05 | - | - | - | - | - | - |
| Base (Y) or (Y') | Y-1 | - | - | - | - | - | - | - | - | - | - |
| | Y-2 | - | - | - | - | - | - | - | - | - | - |
| | Y-3 | - | - | - | | 10.0 | - | - | 10.0 | 10.0 | 10.0 |
| | Y-4 | 5.0 | 5.0 | 5.0 | 5.0 | - | 10.0 | - | - | - | - |
| | Y-5 | - | - | - | - | - | - | 10.0 | - | - | - |
| | Y-6 | - | - | - | - | - | - | - | - | - | - |
| | Y-7-1 | - | - | - | - | - | - | - | - | - | - |

(continued)

| | | | Example | | | | | | | | | |
|---|---|---|---|---|---|---|---|---|---|---|---|---|
| | | | 21 | 22 | 23 | 24 | 25 | 26 | 27 | 28 | 29 | 30 |
| | | Y-7-2 | - | - | - | - | - | - | - | - | - | - |
| | | Y-7-3 | - | - | - | - | - | - | - | - | - | - |
| | | Y-1 | - | - | - | - | - | - | - | - | - | - |
| | | Y-7-4 | - | - | - | - | - | - | - | - | - | - |
| | | Y-8 | - | - | - | - | - | - | - | - | - | - |
| Volatile component | | Ion-exchanged water | 92.00 | 92.00 | 94.00 | 91.95 | 87.00 | 87.00 | 87.00 | 87.00 | 87.00 | 87.00 |
| Viscosity of microbiota improvina aaent (Pa·s) | | | 18.0 | 17.9 | 17.0 | 18.1 | 20 | 19.3 | 42 | 20 | 20 | 20 |
| Worked penetration of microbiota improving agent | | | 280 | 282 | 285 | 280 | 270 | 274 | 260 | 251 | 249 | 250 |
| pH of solution having concentration of 1% by weight of microbiota improving agent prepared by dilution with water | | | 7.1 | 7.1 | 6.5 | 7.2 | 7.1 | 7.2 | 6.8 | 7,1 | 7.2 | 6.9 |
| Confirmation test for selective inhibition of bacterial growth | Pathogenic bacteria | *Porphyromonas gingivalis* | 0.05 | 0.07 | 0.31 | 0.05 | 0.05 | 0.05 | 0.05 | 0.06 | 0.05 | 0.05 |
| | | *Fusobacterium nucleatum* | 0.05 | 0.09 | 0.22 | 0.05 | 0.05 | 0.05 | 0.05 | 0.05 | 0.05 | 0.05 |
| | | *Prevotella intermedia* | 0.06 | 0.06 | 0.17 | 0.05 | 0.06 | 0.05 | 0.05 | 0.05 | 0.06 | 0.05 |
| | | *Trichophyton rubrum* | 0.05 | 0.07 | 0.22 | 0.05 | 0.06 | 0.05 | 0.05 | 0.05 | 0.06 | 0.05 |
| | | *Trichophyton mentagrophytes* | 0.06 | 0.11 | 0.24 | 0.05 | 0.06 | 0.05 | 0.05 | 0.05 | 0.07 | 0.05 |
| | | *Stapjylococcus aureus* | 0.06 | 0.21 | 0.31 | 0.05 | 0.06 | 0.05 | 0.05 | 0.05 | 0.06 | 0.05 |
| | | *Candida albicans* | 0.07 | 0.09 | 0.41 | 0.05 | 0.05 | 0.05 | 0.05 | 0.05 | 0.05 | 0.05 |
| | | *Candida alabrata* | 0.05 | 0.10 | 0.21 | 0.05 | 0.05 | 0.05 | 0.05 | 0.05 | 0.05 | 0.05 |

(continued)

| | | | Example | | | | | | | | | |
|---|---|---|---|---|---|---|---|---|---|---|---|---|
| | | | 21 | 22 | 23 | 24 | 25 | 26 | 27 | 28 | 29 | 30 |
| | Non-pathogenic bacteria | *Streptococcus salivarius* | 1.0 | 1.0 | 1.0 | 0.1 | 1.0 | 0.9 | 1.0 | 0.9 | 1.0 | 1.0 |
| | | *Staphylococcus epidermidis* | 1.0 | 1.0 | 1.0 | 0.1 | 1.0 | 1.0 | 1.0 | 0.9 | 1.0 | 0.9 |
| Inhibition assay of protease production by pathogenic bacteria | | *Porphyromonas gingivalis* | 0.001 | 0.001 | 0.01 | 0.001 | 0.001 | 0.001 | 0.001 | 0.001 | 0.001 | 0.001 |
| | | *Fusobacterium nucleatum* | 0.002 | 0.001 | 0.003 | 0.001 | 0.002 | 0.001 | 0.001 | 0.001 | 0.002 | 0.001 |
| | | *Prevotella intermedia* | 0.001 | 0.001 | 0.002 | 0.001 | 0.001 | 0.001 | 0.001 | 0.001 | 0.001 | 0.001 |
| Persistence evaluation | Pathogenic bacteria | *Porphyromonas gingivalis* | ○ | △ | △ | ○ | ○ | ○ | ○ | ○ | ○ | ○ |
| | | *Fusobacterium nucleatum* | ○ | △ | △ | ○ | ○ | ○ | ○ | ○ | ○ | ○ |
| | | *Prevotella intermedia* | ○ | △ | △ | ○ | ○ | ○ | ○ | ○ | ○ | ○ |
| | | *Trichophyton rubrum* | ○ | △ | △ | ○ | ○ | ○ | ○ | ○ | ○ | ○ |
| | | *Trichoohyton mentagrophytes* | ○ | △ | △ | ○ | ○ | ○ | ○ | ○ | ○ | ○ |
| | | *Staphylococcus aureus* | ○ | △ | △ | ○ | ○ | ○ | ○ | ○ | ○ | ○ |
| | | *Candida albicans* | ○ | △ | △ | ○ | ○ | ○ | ○ | ○ | ○ | ○ |
| | | *Candida alabrata* | ○ | △ | △ | ○ | ○ | ○ | ○ | ○ | ○ | ○ |
| | Non-pathogenic bacteria | *Streptococcus salivarius* | ◎ | ◎ | ◎ | ○ | ◎ | ◎ | ◎ | ◎ | ◎ | ◎ |
| | | *Staphylococcus epidermidis* | ◎ | ◎ | ◎ | ○ | ◎ | ◎ | ◎ | ◎ | ◎ | ◎ |
| Spreadability evaluation | | | ○ | ○ | ○ | ○ | ○ | ○ | ○ | ○ | ○ | ○ |

[Table 1-4]

| | | Example | | | | | | | | | |
|---|---|---|---|---|---|---|---|---|---|---|---|
| | | 31 | 32 | 33 | 34 | 35 | 36 | 37 | 38 | 39 | 40 |
| Substance (X) or (X') | X-1 | - | 3.0 | 3.0 | 3.0 | 3.0 | 3.0 | 3.0 | 3.0 | 3.0 | 3.0 |
| | X-2 | - | - | - | - | - | - | - | - | - | - |
| | X-3 | - | - | - | - | - | - | - | - | - | - |
| | X-4 | - | - | - | - | - | - | - | - | - | - |
| | X-5 | 3.0 | - | - | - | - | - | - | - | - | - |
| | X-6 | - | - | - | - | - | - | - | - | - | - |
| | X-7 | - | - | - | - | - | - | - | - | - | - |
| | X-8 | - | - | - | - | - | - | - | - | - | - |
| | X-9 | - | - | - | - | - | - | - | - | - | - |
| | X-10 | - | - | - | - | - | - | - | - | - | - |
| | X-11 | - | - | - | - | - | - | - | - | - | - |
| | X-12 | - | - | - | - | - | - | - | - | - | - |
| | X-13 | - | - | - | - | - | - | - | - | - | - |
| | X-14 | - | - | - | - | - | - | - | - | - | - |
| | X-15 | - | - | - | - | - | - | - | - | - | - |
| | X-16 | - | - | - | - | - | - | - | - | - | - |
| | X'-1 | - | - | - | - | - | - | - | - | - | - |
| | X'-2 | - | - | - | - | - | - | - | - | - | - |
| Base (Y) or (Y') | Y-1 | - | - | - | - | - | - | - | - | - | - |
| | Y-2 | - | - | - | - | - | - | - | - | 1.0 | - |
| | Y-3 | 10.0 | - | - | - | - | - | - | - | - | - |
| | Y-4 | - | - | - | - | - | 0.1 | 1.0 | 3.0 | - | 0.1 |
| | Y-5 | - | - | - | - | - | - | - | - | - | - |
| | Y-6 | - | 50.0 | - | - | - | - | - | - | - | - |
| | Y-7-1 | - | - | 97.0 - | - | - | 96.9 | 96.0 | 94.0 | 96.0 | - |

(continued)

| | | | Example | | | | | | | | | |
|---|---|---|---|---|---|---|---|---|---|---|---|---|
| | | | 31 | 32 | 33 | 34 | 35 | 36 | 37 | 38 | 39 | 40 |
| | | Y-7-2 | - | - | - | 97.0 | - | - | - | - | - | - |
| | | Y-7-3 | - | - | - | - | 97.0 | - | - | - | - | 96.9 |
| | | Y'-1 | - | - | - | - | - | - | - | - | - | - |
| | | Y-7-4 | - | - | - | - | - | - | - | - | - | - |
| | | Y-8 | - | - | - | - | - | - | - | - | - | - |
| Volatile component | | Ion-exchanged water | 87.00 | 47.00 | - | - | - | - | - | - | - | - |
| Viscosity of microbiota improving agent (Pa·s) | | | 20 | 2.2 | 3500 | 90 | 8.9 | 3400 | 3650 | 3900 | 3600 | 8.6 |
| Worked penetration of microbiota improving agent | | | 251 | 298 | 250 | 301 | 390 | 249 | 239 | 101 | 260 | 392 |
| pH of solution having concentration of 1% by weight of microbiota improving agent prepared by dilution with water | | | 7.0 | 7.1 | 6.5 | 6.8 | 7.0 | 6.6 | 6.9 | 7.0 | 6.8 | 7.0 |
| Confirmation test for selective inhibition of bacterial growth | Pathogenic bacteria | *Porphyromonas oinoivalis* | 0.05 | 0.06 | 0.05 | 0.05 | 0.05 | 0.05 | 0.03 | 0.05 | 0.04 | 0.06 |
| | | *Fusobacterium nucleatum* | 0.05 | 0.05 | 0.05 | 0.05 | 0.05 | 0.05 | 0.04 | 0.05 | 0.05 | 0.05 |
| | | | 0.06 | 0.07 | 0.05 | 0.05 | 0.05 | 0.06 | 0.03 | 0.06 | 0.06 | 0.06 |
| | | *Prevotella intermedia Trichoohyton rubrum* | 0.05 | 0.07 | 0.05 | 0.05 | 0.05 | 0.05 | 0.02 | 0.05 | 0.03 | 0.07 |
| | | *Trichoohvton* | 0.06 | 0.05 | 0.05 | 0.06 | 0.05 | 0.06 | 0.03 | 0.06 | 0.04 | 0.06 |
| | | *Staohylococcus aureus* | 0.06 | 0.07 | 0.06 | 0.05 | 0.05 | 0.06 | 0.03 | 0.06 | 0.04 | 0.05 |
| | | *Candida albicans* | 0.05 | 0.07 | 0.05 | 0.05 | 0.07 | 0.05 | 0.05 | 0.07 | 0.05 | 0.05 |
| | | *Candida alabrata* | 0.05 | 0.07 | 0.05 | 0.05 | 0.08 | 0.05 | 0.05 | 0.08 | 0.05 | 0.05 |
| | Non-pathogenic bacteria | *Streptococcus salivarius* | 1.1 | 0.9 | 1.1 | 1.0 | 1.0 | 1.1 | 1.1 | 1.1 | 1.0 | 1.0 |
| | | *Staphylococcus epidermidis* | 1.0 | 1.0 | 0.9 | 1.0 | 0.9 | 1.0 | 1.0 | 1.0 | 0.9 | 1.0 |

(continued)

| | | | Example | | | | | | | | | |
|---|---|---|---|---|---|---|---|---|---|---|---|---|
| | | | 31 | 32 | 33 | 34 | 35 | 36 | 37 | 38 | 39 | 40 |
| Inhibition assay of protease production by pathogenic bacteria | | *Porphyromonas gingivalis* | 0.001 | 0.002 | 0.001 | 0.001 | 0.001 | 0.001 | 0.001 | 0.001 | 0.001 | 0.001 |
| | | *Fusobacterium nucleatum* | 0.002 | 0.001 | 0.001 | 0.001 | 0.001 | 0.002 | 0.001 | 0.002 | 0.001 | 0.002 |
| | | *Prevotella intermedia* | 0.001 | 0.001 | 0.001 | 0.001 | 0.002 | 0.001 | 0.001 | 0.001 | 0.001 | 0.001 |
| Persistence evaluation | Pathogenic bacteria | *Porphyromonas gingivalis* | ○ | △ | ○ | ○ | △ | ○○ | ○○ | ○○ | ○○ | ○○ |
| | | *Fusobacterium nucleatum* | ○ | △ | ○ | ○ | △ | ○○ | ○○ | ○○ | ○○ | ○○ |
| | | *Prevotella intermedia* | ○ | △ | ○ | ○ | △ | ○○ | ○○ | ○○ | ○○ | ○○ |
| | | *Trichophyton rubrum* | ○ | △ | ○ | ○ | △ | ○○ | ○○ | ○○ | ○○ | ○○ |
| | | *Trichophyton* | ○ | △ | ○ | ○ | △ | ○○ | ○○ | ○○ | ○○ | ○○ |
| | | *Staphylococcus aureus* | ○ | △ | ○ | ○ | △ | ○○ | ○○ | ○○ | ○○ | ○○ |
| | | *Candida albicans* | ○ | △ | ○ | ○ | △ | ○○ | ○○ | ○○ | ○○ | ○○ |
| | | *Candida alabrata* | ○ | △ | ○ | ○ | △ | ○○ | ○○ | ○○ | ○○ | ○○ |
| | Non-pathogenic bacteria | *Streptococcus salivarius* | ○○ | ○○ | ○○ | ○○ | ○○ | ○○ | ○○ | ○○ | ○○ | ○○ |
| | | *Staphylococcus epidermidis* | ○○ | ○○ | ○○ | ○○ | ○○ | ○○ | ○○ | ○○ | ○○ | ○○ |
| Spreadability evaluation | | | ○ | ○ | ○○ | ○ | ○ | ○○ | ○○ | ○ | ○○ | ○ |

[Table 1-5]

| | | Example | | | | Comparative Example | | | | | |
|---|---|---|---|---|---|---|---|---|---|---|---|
| | | 41 | 42 | 43 | 44 | 1 | 2 | 3 | 4 | 5 | 6 |
| Substance (X) or (X') | X-1 | 3.0 | 3.0 | 3.0 | 3.0 | - | - | - | - | - | - |
| | X-2 | - | - | - | - | - | - | - | - | - | - |
| | X-3 | - | - | - | - | - | - | - | - | - | - |
| | X-4 | - | - | - | - | - | - | - | - | - | - |
| | X-5 | - | - | - | - | - | - | - | - | - | - |
| | X-6 | - | - | - | - | - | - | - | - | - | - |
| | X-7 | - | - | - | - | - | - | - | - | - | - |
| | X-8 | - | - | - | - | - | - | - | - | - | - |
| | X-9 | - | - | - | - | - | - | - | - | - | - |
| | X-10 | - | - | - | - | - | - | - | - | - | - |
| | X-11 | - | - | - | - | - | - | - | - | - | - |
| | X-12 | - | - | - | - | - | - | - | - | - | - |
| | X-13 | - | - | - | - | - | - | - | - | - | - |
| | X-14 | - | - | - | - | - | - | - | - | - | - |
| | X-15 | - | - | - | - | - | - | - | - | - | - |
| | X-16 | - | - | - | - | - | - | - | - | - | - |
| | X'-1 | - | - | - | - | - | - | - | - | - | - |
| | X'-2 | - | - | - | - | - | - | - | - | - | - |
| Base (Y) or (Y') | Y-1 | - | - | - | - | 2.0 | - | - | - | - | - |
| | Y-2 | - | - | - | - | - | 2.0 | - | - | - | - |
| | Y-3 | 0.1 | 1.0 | 5.0 | 0.1 | - | - | 10.0 | - | - | - |
| | Y-4 | - | - | - | - | - | - | - | 5.0 | 10.0 | - |
| | Y-5 | - | - | - | - | - | - | - | - | - | 10.0 |
| | Y-6 | - | - | - | - | - | - | - | - | - | - |
| | Y-7-1 | 96.9 | 96.0 | 92.0 | - | - | - | - | - | - | - |

(continued)

| | | | Example | | | | Comparative Example | | | | | |
|---|---|---|---|---|---|---|---|---|---|---|---|---|
| | | | 41 | 42 | 43 | 44 | 1 | 2 | 3 | 4 | 5 | 6 |
| | | Y-7-2 | - | - | - | - | - | - | - | - | - | - |
| | | Y-7-3 | - | - | - | 96.9 | - | - | - | - | - | - |
| | | Y'-1 | - | - | - | - | - | - | - | - | - | - |
| | | Y-7-4 | - | - | - | - | - | - | - | - | - | - |
| | | Y-8 | - | - | - | - | - | - | - | - | - | - |
| Volatile component | | Ion-exchanged water | - | - | - | - | 98.00 | 98.00 | 90.00 | 95,00 | 90,00 | 90.00 |
| Viscosity of microbiota improving agent (Pa·s) | | | 3400 | 3650 | 3900 | 9.0 | 5.5 | 13.1 | 20 | 17.9 | 19.3 | 42 |
| Worked penetration of microbiota improving agent | | | 249 | 239 | 101 | 391 | 291 | 280 | 270 | 285 | 273 | 261 |
| pH of solution having concentration of 1% by weight of microbiota improving agent prepared by dilution with water | | | 6.6 | 6.9 | 7.0 | 7.0 | 6.9 | 7.0 | 7.1 | 7.2 | 7.2 | 6.8 |
| Confirmation test for selective inhibition of bacterial growth | Pathogenic bacteria | *Porohyromonas ai-naivalis* | 0.05 | 0.03 | 0.05 | 0.06 | 1.0 | 0.9 | 0.9 | 0.9 | 0.9 | 0.9 |
| | | *Fusobacterium nucleatum* | 0.05 | 0.04 | 0.05 | 0.05 | 1.0 | 1.0 | 1.0 | 1.0 | 1.0 | 1.0 |
| | | *Prevotella intermedia* | 0.06 | 0.03 | 0.06 | 0.06 | 1.0 | 1.0 | 1.0 | 0.9 | 0.9 | 1.0 |
| | | *Trichoohvton ru-brum* | 0.05 | 0.02 | 0.05 | 0.07 | 1.0 | 0.9 | 1.1 | 0.9 | 0.9 | 0.9 |
| | | *Trichoohyton mentagrophytes* | 0.06 | 0.03 | 0.06 | 0.06 | 1.0 | 0.9 | 0.9 | 0.9 | 0.9 | 0.9 |
| | | *Staphylococcus aureus* | 0.06 | 0.03 | 0.06 | 0.05 | 1.0 | 1.1 | 0.9 | 0.9 | 0.9 | 0.9 |
| | | *Candida albicans* | 0.05 | 0.05 | 0.08 | 0.05 | 1.0 | 1.0 | 1.0 | 1.0 | 0.9 | 0.9 |
| | | *Candida alabrata* | 0.05 | 0.05 | 0.09 | 0.05 | 0.9 | 1.0 | 1.0 | 1.0 | 1.0 | 1.0 |

(continued)

| | | | Example | | | | Comparative Example | | | | | |
|---|---|---|---|---|---|---|---|---|---|---|---|---|
| | | | 41 | 42 | 43 | 44 | 1 | 2 | 3 | 4 | 5 | 6 |
| | Non-pathogenic bacteria | *Streptococcus salivarius* | 1.1 | 1.1 | 1.1 | 1.0 | 1.0 | 0.9 | 0.9 | 0.9 | 0.9 | 1.0 |
| | | *Staphylococcus epidermidis* | 1.0 | 1.0 | 1.0 | 1.0 | 1.0 | 1.0 | 1.0 | 1.0 | 1.0 | 1.0 |
| Inhibition assay of protease production by pathogenic bacteria | | *Porphyromonas gingivalis* | 0.001 | 0.001 | 0.001 | 0.001 | 1.0 | 1.0 | 1.1 | 1.0 | 1.0 | 1.0 |
| | | *Fusobacterium nucleatum* | 0.002 | 0.001 | 0.002 | 0.002 | 1.0 | 1.0 | 1.0 | 1.0 | 1.0 | 1.0 |
| | | *Prevotella intermedia* | 0.001 | 0.001 | 0.001 | 0.001 | 1.0 | 0.9 | 1.0 | 1.0 | 1.0 | 1.0 |
| Persistence evaluation | Pathogenic bacteria | *Porohyromonas ainaivalis* | ○○ | ○○ | ○○ | ○○ | × | × | × | × | × | × |
| | | *Fusobacterium nucleatum* | ○○ | ○○ | ○○ | ○○ | × | × | × | × | × | × |
| | | *Prevotella intermedia* | ○○ | ○○ | ○○ | ○○ | × | × | × | × | × | × |
| | | *Trichophyton rubrum* | ○○ | ○○ | ○○ | ○○ | × | × | × | × | × | × |
| | | *Trichoohyton mentagrophytes* | ○○ | ○○ | ○○ | ○○ | × | × | × | × | × | × |
| | | *Staphylococcus aureus* | ○○ | ○○ | ○○ | ○○ | × | × | × | × | × | × |
| | | *Candida albicans* | ○○ | ○○ | ○○ | ○○ | × | × | × | × | × | × |
| | | *Candida alabrata* | ○○ | ○○ | ○○ | ○○ | × | × | × | × | × | × |
| | Non-pathogenic bacteria | *Streptococcus salivarius* | ○○ | ○○ | ○○ | ○○ | ○○ | ○○ | ○○ | ○○ | ○○ | ○○ |
| | | *Staphylococcus epidermidis* | ○○ | ○○ | ○○ | ○○ | ○○ | ○○ | ○○ | ○○ | ○○ | ○○ |
| Spreadability evaluation | | | ○○ | ○○ | ○ | ○ | ○ | ○ | ○ | ○ | ○ | ○ |

[Table 1-6]

| | | Comparative Example | | | | | | | | | |
|---|---|---|---|---|---|---|---|---|---|---|---|
| | | 7 | 8 | 9 | 10 | 11 | 12 | 13 | 14 | 15 | 16 |
| Substance (X) or (X') | X-1 | - | - | - | - | 3.0 | 3.0 | 3.0 | 3.0 | - | - |
| | X-2 | - | - | - | - | - | - | - | - | - | - |
| | X-3 | - | - | - | - | - | - | - | - | - | - |
| | X-4 | - | - | - | - | - | - | - | - | - | - |
| | X-5 | - | - | - | - | - | - | - | - | - | - |
| | X-6 | - | - | - | - | - | - | - | - | - | - |
| | X-7 | - | - | - | - | - | - | - | - | - | - |
| | X-8 | - | - | - | - | - | - | - | - | - | - |
| | X-9 | - | - | - | - | - | - | - | - | - | - |
| | X-10 | - | - | - | - | - | - | - | - | - | - |
| | X-11 | - | - | - | - | - | - | - | - | - | - |
| | X-12 | - | - | - | - | - | - | - | - | - | - |
| | X-13 | - | - | - | - | - | - | - | - | - | - |
| | X-14 | - | - | - | - | - | - | - | - | - | - |
| | X-15 | - | - | - | - | - | - | - | - | - | - |
| | X-16 | - | - | - | - | - | - | - | - | - | - |
| | X'-1 | - | - | - | - | - | - | - | - | 3.0 | - |
| | X'-2 | - | - | - | - | - | - | - | - | - | 3.0 |
| Base (Y) or (Y') | Y-1 | - | - | - | - | - | - | - | - | - | - |
| | Y-2 | - | - | - | - | - | - | - | - | - | - |
| | Y-3 | - | - | - | - | - | 1.0 | - | - | 10.0 | 10.0 |
| | Y-4 | - | - | - | - | - | - | - | - | - | - |
| | Y-5 | - | - | - | - | - | - | - | - | - | - |
| | Y-6 | 50.0 | - | - | - | - | - | - | - | - | - |
| | Y-7-1 | - | 1000 | - | - | - | - | - | - | - | - |

(continued)

| | | | Comparative Example | | | | | | | | | |
|---|---|---|---|---|---|---|---|---|---|---|---|---|
| | | | 7 | 8 | 9 | 10 | 11 | 12 | 13 | 14 | 15 | 16 |
| | | Y-7-2 | - | - | 100.0 | - | - | - | - | - | - | - |
| | | Y-7-3 | - | - | - | 1000 | - | - | - | - | - | - |
| | | Y'-1 | - | - | - | - | 97.0 | - | - | - | - | - |
| | | Y-7-4 | - | - | - | - | - | - | 97.0 | - | - | - |
| | | Y-8 | - | - | - | - | - | - | - | - | - | - |
| Volatile component | | Ion-exchanced water | 50.00 | - | - | - | - | 96.00 | - | 97.00 | 87.00 | 87.00 |
| Viscosity of microbiota improving agent (Pa·s) | | | 2.2 | 3500 | 90 | 8.9 | 100 | 1.1 | 4500 | Less than 1 | 20 | 18 |
| Worked penetration of microbiota improving agent | | | 298 | 282 | 257 | 250 | 250 | 450 | 95 | | 271 | 265 |
| pH of solution having concentration of 1% by weight of microbiota improving agent prepared by dilution with water | | | 7.1 | 7.0 | 6.8 | 6.5 | 6.8 | 7.0 | 6.9 | 7.0 | 6.9 | 7.3 |
| Confirmation test for selective inhibition of bacterial growth | Pathogenic bacteria | *Porphyromonas gingivalis* | 1.0 | 0.9 | 1.0 | 0.9 | 0.1 | 0.1 | 1.0 | 0.1 | 0.03 | 0.04 |
| | | *Fusobacterium nucleatum* | 1.0 | 0.9 | 1.0 | 1.0 | 0.1 | 0.1 | 1.1 | 0.1 | 0.04 | 0.05 |
| | | *Prevotella intermedia* | 1.0 | 1.0 | 1.0 | 1.1 | 0.1 | 0.1 | 1.0 | 0.1 | 0.03 | 0.03 |
| | | *Trichophyton rubrum* | 0.9 | 1.0 | 0.9 | 0.9 | 0.1 | 0.08 | 0.9 | 0.09 | 0.05 | 0.05 |
| | | *Trichophyton mentagrophytes* | 1.0 | 0.9 | 0.9 | 1.0 | 0.1 | 0.09 | 0.8 | 0.11 | 0.03 | 0.03 |
| | | *Staphylococcus aureus* | 1.0 | 0.9 | 0.9 | 1.1 | 0.1 | 0.1 | 1.0 | 0.09 | 0.03 | 0.03 |
| | | *Candida albicans* | 1.0 | 1.0 | 0.9 | 0.9 | 0.1 | 0.1 | 0.9 | 0.1 | 0.04 | 0.05 |
| | | *Candida alabrata* | 0.9 | 1.0 | 1.0 | 1.0 | 0.1 | 0.09 | 1.0 | 0.1 | 0.04 | 0.05 |

(continued)

| | | | Comparative Example | | | | | | | | | |
|---|---|---|---|---|---|---|---|---|---|---|---|---|
| | | | 7 | 8 | 9 | 10 | 11 | 12 | 13 | 14 | 15 | 16 |
| | Non-pathogenic bacteria | *Streptococcus salivarius* | 0.9 | 0.9 | 0.9 | 1.0 | 1.1 | 0.1 | 1.0 | 0.1 | 0.02 | 0.03 |
| | | *Staphylococcus epidermidis* | 1.0 | 1.0 | 1.0 | 1.0 | 0.9 | 0.1 | 0.9 | 0.1 | 0.03 | 0.05 |
| Inhibition assay of protease production by pathogenic bacteria | | *Porphyromonas gingivalis* | 1.0 | 1.1 | 1.0 | 1.0 | 0.001 | 0.001 | 1.0 | 0.002 | 0.001 | 0.002 |
| | | *Fusobacterium nucleatum* | 1.0 | 1.0 | 1.0 | 0.9 | 0.001 | 0.001 | 0.9 | 0.001 | 0.001 | 0.001 |
| | | *Prevotella intermedia* | 1.0 | 1.1 | 1.0 | 1.0 | 0.001 | 0.001 | 1.0 | 0.001 | 0.001 | 0.001 |
| Persistence evaluation | Pathogenic bacteria | *Porphyromonas gingivalis* | × | × | × | × | × | × | × | × | ○ | ○ |
| | | *Fusobacterium nucleatum* | × | × | × | × | × | × | × | × | ○ | ○ |
| | | *Prevotella intermedia* | × | × | × | × | × | × | × | × | ○ | ○ |
| | | *Trichophyton rubrum* | × | × | × | × | × | × | × | × | ○ | ○ |
| | | *Trichophyton mentagrophytes* | × | × | × | × | × | × | × | × | ○ | ○ |
| | | *Staohylococcus aureus* | × | × | × | × | × | × | × | × | ○ | ○ |
| | | *Candida albicans* | × | × | × | × | × ○○ | × | × | × | ○ | ○ |
| | | *Candida alabrata* | × | × | × | × | × | × | × | × | ○ | ○ |
| | Non-pathogenic bacteria | *Streptococcus salivarius* | ○○ | ○○ | ○○ | ○○ | | × | ○○ | × | × | × |
| | | *Staphylococcus epidermidis* | ○○ | ○○ | ○○ | ○○ | ○○ | × | ○○ | × | × | × |
| Spreadability evaluation | | | ○ | ○○ | ○ | ○ | × | ○ | △ | × | ○ | ○ |

[Table 2]

| | | HLB value of non-volatile component | Weight average molecular weight of non-volatile component | Viscosity (mPa·s) of solution having concentration of 1% by weight of non-volatile component prepared by dilution with water |
|---|---|---|---|---|
| Base (Y) or (Y') | Y-1 | 27.1 | 49,000 | 200 |
| | Y-2 | 44.0 | 200,000 | 1,000 |
| | Y-3 | 58.2 | 100,000 | 500 |
| | Y-4 | 34.6 | 2,000,000 | 1,500 |
| | Y-5 | 30.0 | 1,000,000 | 800 |
| | Y-6 | 18.9 | 20,000 | 1,000 |
| | Y-7-1 | 7.8 | 28,000 | 10 |
| | Y-7-2 | 7.8 | 28,000 | 10 |
| | Y-7-3 | 7.8 | 28,000 | 10 |
| | Y'-1 | 0 | 300 | 0.8 |
| | Y-7-4 | 7.8 | 28,000 | 10.0 |
| | Y-8 | 34.3 | 140,000 | 1,000 |

[Table 3]

| | | Amount of magnesium (g) captured per 100 g of substance (X) |
|---|---|---|
| Substance (X) | X-1 | 5 |
| | X-2 | 3 |
| | X-3 | 3 |
| | X-4 | 2 |
| | X-5 | 2 |
| | X-6 | 2 |
| | X-7 | 2 |
| | X-8 | 15 |
| | X-9 | 20 |
| | X-10 | 30 |
| | X-11 | 15 |
| | X-12 | 10 |
| | X-13 | 8 |
| | X-14 | 2 |
| | X-15 | 9 |
| | X-16 | 5 |

As shown in Tables 1-1 to 1-6, in Examples 1 to 44 containing the substances (X) and (Y), the growth of pathogenic bacteria was inhibited selectively, whereas the growth of non-pathogenic bacteria was not inhibited, thus achieving an ideal microbiota balance, compared with Comparative Examples 1 to 16. Therefore, the microbiota improvement effect was confirmed in Examples 1 to 44.

Furthermore, in Comparative Examples 15 and 16 in which a common antibacterial agent (benzalkonium chloride or cetylpyridinium chloride) was used, the growth of not only pathogenic bacteria but also non-pathogenic bacteria were inhibited. On the other hand, in Examples 1 to 44, the growth of non-pathogenic bacteria was maintained, suggesting the effect of selectively inhibiting pathogenic bacteria.

Furthermore, in Examples 1 to 44, an inhibitory effect on the production of toxins (proteases) by pathogenic bacteria was confirmed. Also, the long-term persistence of the microbiota improving effect was confirmed compared with Comparative Examples 11, 12, and 14 in which a protease production inhibitory effect is exhibited.

Therefore, the microbiota improving agents of the present invention were found to have the effect of selectively inhibiting the growth of pathogenic bacteria and inhibiting toxin production by pathogenic bacteria without affecting non-pathogenic bacteria and to allow the effect to persist for a long period of time. Once the microbiota improving agent of the present invention is applied to the affected area, it can exhibit a microbiota balance improving effect for a long period of time. Thus, the microbiota improving agents of the present invention are particularly useful as a microbiota improving agent.

## Claims

1. A microbiota improving agent for improving at least one of mucosal microbiota, skin microbiota, or nail microbiota, the microbiota improving agent comprising:

   a substance (X); and
   a base (Y) containing a non-volatile component,
   the substance (X) including at least one selected from the group consisting of
   a compound (X1) having two or more hydroxy groups contained in at least one oxoacid group and a salt of the compound (X1),
   a compound (X2) having one hydroxy group contained in an oxoacid group and a salt of the compound (X2),
   a heterocyclic compound (X3) having no oxoacid group and a salt of the compound (X3), and
   a ketone (X4) having no oxoacid group,
   wherein in the compound (X1), a total mass percentage of the at least one oxoacid group, a hydroxy group not contained in the oxoacid group, a carbonyl group not contained in the oxoacid group, carbon atoms contained in an imidazole skeleton and a pyridine skeleton, and nitrogen atoms contained in the imidazole skeleton and the pyridine skeleton is 45% by mass or more based on a molecular weight of the compound (X1),
   the compound (X2) has an atom having an unshared electron pair, the atom excluding atoms contained in the oxoacid group and atoms contained in an amino group not constituting an imidazole skeleton and in an amino group not constituting a pyridine skeleton,
   in the compound (X2), a total mass percentage of the oxoacid group, a hydroxy group not contained in the oxoacid group, a carbonyl group not contained in the oxoacid group, carbon atoms contained in an imidazole skeleton and a pyridine skeleton, and nitrogen atoms contained in the imidazole skeleton and the pyridine skeleton is 55% by mass or more based on a molecular weight of the compound (X2),
   the heterocyclic compound (X3) has an imidazole skeleton or a pyridine skeleton and at least one of a hydroxy group or a carbonyl group, or has two or more skeletons selected from the group consisting of an imidazole skeleton and a pyridine skeleton,
   the ketone (X4) has a linear $\beta$-diketone structure,
   the substance (X) has a molecular weight of 550 or less,
   the non-volatile component has an HLB value of 7 or more, and
   the microbiota improving agent satisfies the following conditions (i) and (ii):

      (i) the microbiota improving agent has a worked penetration of 100 to 400 as measured in accordance with JIS K 2220; and
      (ii) the microbiota improving agent has a viscosity at 37°C of 1 to 4,000 Pa·s as measured with a cone-and-plate rheometer.

2. The microbiota improving agent according to claim 1,
   wherein a solution having a concentration of 1% by weight of the microbiota improving agent prepared by diluting the microbiota improving agent with water has a pH of 6 to 8.

3. The microbiota improving agent according to claim 1 or 2,
   wherein a weight percentage of the substance (X) is 0.01 to 5% by weight based on a weight of the microbiota improving agent.

**INTERNATIONAL SEARCH REPORT**

International application No. **PCT/JP2023/015811**

**A. CLASSIFICATION OF SUBJECT MATTER**

***A61P 1/02***(2006.01)i; ***A61Q 3/00***(2006.01)i; ***A61Q 11/00***(2006.01)i; ***A61Q 19/00***(2006.01)i; ***A61K 9/06***(2006.01)i; ***A61K 9/08***(2006.01)i; ***A61K 47/38***(2006.01)i; ***A61K 33/00***(2006.01)i; ***A61K 33/24***(2019.01)i; ***A61K 33/42***(2006.01)i; ***A23L 29/206***(2016.01)i; ***A23L 29/269***(2016.01)i; ***A23L 29/288***(2016.01)i; ***A23L 33/10***(2016.01)i; ***A61K 8/19***(2006.01)i; ***A61K 8/24***(2006.01)i; ***A61K 8/35***(2006.01)i; ***A61K 8/36***(2006.01)i; ***A61K 8/365***(2006.01)i; ***A61K 8/73***(2006.01)i; ***A61K 31/12***(2006.01)i; ***A61K 31/121***(2006.01)i; ***A61K 31/19***(2006.01)i

FI: A61K31/19; A61Q11/00; A61Q3/00; A61K8/19; A61K8/35; A61K8/24; A61K8/73; A61P1/02; A61K33/00; A61K31/12; A61K33/42; A61K31/121; A61K9/06; A61K9/08; A61K47/38; A23L33/10; A23L29/288; A23L29/269; A23L29/206; A61K33/24; A61K8/36; A61Q19/00; A61K8/365

According to International Patent Classification (IPC) or to both national classification and IPC

**B. FIELDS SEARCHED**

Minimum documentation searched (classification system followed by classification symbols)

A61P1/02; A61Q3/00; A61Q11/00; A61Q19/00; A61K9/06; A61K9/08; A61K47/38; A61K33/00; A61K33/24; A61K33/42; A23L29/206; A23L29/269; A23L29/288; A23L33/10; A61K8/19; A61K8/24; A61K8/35; A61K8/36; A61K8/365; A61K8/73; A61K31/12; A61K31/121; A61K31/19

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2005-298357 A (LION CORP) 27 October 2005 (2005-10-27)<br>claims, paragraphs [0007], [0016]-[0018], [0021]-[0022], [0028], example 1 | 1-3 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **10 July 2023** | **18 July 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

INTERNATIONAL SEARCH REPORT

International application No.
PCT/JP2023/015811

**Box No. III Observations where unity of invention is lacking (Continuation of item 3 of first sheet)**

This International Searching Authority found multiple inventions in this international application, as follows:

(Invention 1) Invention selecting (X1) as (X) component in claims 1-3 Claim 1 sets forth the invention relating to a "microbial flora improving agent for improving microbial flora in the mucosa, skin, and/or nails, the agent containing: a substance (X) that is at least one selected from the group consisting of a compound (X1) and a salt thereof having two or more hydroxy groups contained in an oxo acid group, a compound (X2) and a salt thereof having one hydroxy group contained in an oxo acid group, a heterocyclic compound (X3) and a salt thereof having no oxo acid group, and a ketone (X4) having no oxo acid group; and a base (Y) containing a non-volatile component, wherein the ratio of the total mass of the oxo acid group of the compound (X1), the hydroxy group not contained in the oxo acid group, a carbonyl group not contained in the oxo acid group, the carbon atoms contained in an imidazole skeleton and a pyridine skeleton, and the nitrogen atoms contained in the imidazole skeleton and the pyridine skeleton is 45 mass% or more based on the molecular weight of the compound (X1), and the compound (X2) has an atom having an unshared electron pair (with the proviso that the atom contained in the oxo acid group, the amino group not constituting the imidazole skeleton, and atoms contained in the amino group not constituting the pyridine skeleton are excluded), the ratio of the total mass of the oxo acid group of the compound (X2), the hydroxy group not contained in the oxo acid group, the carbonyl group not contained in the oxo acid group, the carbon atoms contained in the imidazole skeleton and the pyridine skeleton, and the nitrogen atoms contained in the imidazole skeleton and the pyridine skeleton is 55 mass% or more based on the molecular weight of the compound (X2), the heterocyclic compound (X3) has an imidazole skeleton or a pyridine skeleton, and a hydroxy group and/or a carbonyl group, or at least two skeletons selected from the imidazole skeleton and the pyridine skeleton, the ketone (X4) has a linear β-diketone structure, the molecular weight of the substance (X) is 550 or less, the HLB value of the non-volatile component is 7 or more, and the following conditions (i) and (ii) are met: (i) the worked penetration of the microbial flora improving agent as measured according to JIS K 2220 is 100-400; and (ii) the viscosity at 37 °C of the microbial flora improving agent as measured by a cone plate-type rotational viscometer is 1-4,000 Pa·s."

The invention first set forth in the present application is the invention of a "microbial flora improving agent that selects (X1) as a component (X) and contains a base (Y) containing a non-volatile component," of the invention in claim 1, and the "microbial flora improving agent containing a substance (X) selected from the group consisting of (X2) to (X4) other than (X1) as a component (X) and a base (Y) containing a non-volatile component" of said invention and the invention in claim 1 shares the common technical feature of the "microbial flora improving agent for improving microbial flora in the mucosa, skin, and/or nails, the agent containing a substance (X) and a base (Y) containing a non-volatile component, wherein the molecular weight of the substance (X) is 550 or less, the HLB value of the non-volatile component is 7 or more, and the following conditions (i) and (ii) are met: (i) the worked penetration of the microbial flora improving agent as measured according to JIS K 2220 is 100-400; and (ii) the viscosity at 37 °C of the microbial flora improving agent as measured by a cone plate-type rotational viscometer is 1-4,000 Pa·s."

In addition, the invention selecting (X1) as (X) and the invention selecting (X2) as (X) share the common technical feature of a "microbial flora improving agent for improving microbial flora in the mucosa, skin, and/or nails, the agent containing, as a component (X), a compound and a salt thereof containing a hydroxy group contained in an oxo acid group, and a base (Y) containing a non-volatile component, wherein the molecular weight of the substance (X) is 550 or less, the HLB value of the non-volatile component is 7 or more, and the following conditions (i) and (ii) are met: (i) the worked penetration of the microbial flora improving agent as measured according to JIS K 2220 is 100-400; and (ii) the viscosity at 37 °C of the microbial flora improving agent as measured by a cone plate-type rotational viscometer is 1-4,000 Pa·s."

However, document JP 2005-298357 A (LION CORP) 27 October 2005 (2005-10-27) (claims, paragraphs [0007], [0016]-[0018], [0021]-[0022], [0028], example 1) discloses a composition for oral cavity, the composition suppressing the formation of pathogenic plaque by the sterilization and bacteriostasis of pathogenic bacteria in plaque attached to the oral cavity without affecting the indigenous flora in the oral cavity, and being capable of effectively preventing or treating oral diseases (equivalent to a microbial flora improving agent for improving microbial flora in the oral mucosa), and indicates that the composition for oral cavity can be in the dosage form of dentifrice such as a toothpaste, mouth washes, gum massage cream, local application agent, or the like, and in example 1, a toothpaste contains carboxymethyl cellulose sodium (equivalent to Y) and sodium malate (equivalent to X1). The worked penetration and viscosity of the dentifrice can be said to meet the conditions (i) and (ii) above, and thus the common technical feature cannot be said to be a special technical feature. Furthermore, there are no other same or corresponding special technical features between these inventions.

Therefore, the invention selecting, as the component (X), others except (X1) in claim 1 cannot be classified as invention 1.

INTERNATIONAL SEARCH REPORT

International application No. PCT/JP2023/015811

**Box No. III Observations where unity of invention is lacking (Continuation of item 3 of first sheet)**

In claims 2-3, the invention selecting (X1) as the component (X) is also classified as invention 1.
(Invention 2) Invention selecting (X2) as component (X) in claims 1-3
(Invention 3) Invention selecting (X3) as component (X) in claims 1-3
(Invention 4) Invention selecting (X4) as component (X) in claims 1-3

From the above-described reasons, the inventions respectively selecting (X2), (X3), and (X4) as component (X) in claim 1 are classified as inventions 2-4.

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **Invention selecting (X1) as component (X) in claims 1-3.**

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (January 2015)

**INTERNATIONAL SEARCH REPORT**
**Information on patent family members**

International application No.
**PCT/JP2023/015811**

| Patent document cited in search report | Publication date (day/month/year) | Patent family member(s) | Publication date (day/month/year) |
|---|---|---|---|
| JP 2005-298357 A | 27 October 2005 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 5716173 B

### Non-patent literature cited in the description

- **TAKEHIKO FUJIMOTO**. Introduction to Surfactants (Kaimen Kakuseizai Nyumon). *Sanyo Chemical Industries, Ltd.*, 2007, 212